# EUROPÄISCHE PATENTANMELDUNG

(11) **EP 2 233 067 A1**
(43) Veröffentlichungstag der Anmeldung: **29.09.2010**
(21) Anmeldenummer: 09155892.4
(22) Anmeldetag: 23.03.2009
(51) Int. Cl.: A61B 5/00

(54) **Medizinisches System mit Plug-and-Play-Funktion**

(71) Anmelder: Roche Diagnostics GmbH, 68305 Mannheim (DE); F. Hoffmann-La Roche AG, 4070 Basel (CH)
(72) Erfinder: Rösicke, Bernd, 68305 Mannheim (DE); Bernini, Nicole, 3423 Ersigen (CH); Buck, Harvey, 46256 Indianapolis (US); Froech, Sybille, Dr., 68305 Mannheim (DE); Gaa, Otto, Dr., 67551 Worms (DE); Marquant, Michael, 68309 Mannheim (DE); Rasch-Menges, Jürgen, Dr., 68723 Schwetzingen (DE); Scherer, Jörg, 73432 Aalen (DE); Eberhart, Andreas, 6340 Inwil bei Baar (CH)
(74) Vertreter: Stößel, Matthias

(57) **Zusammenfassung**

Es wird ein medizinisches System (144) vorgeschlagen, welches insbesondere zur Überwachung und/oder Steuerung mindestens einer Körperfunktion eines Benutzers (122) einsetzbar sein kann. Das medizinische System (144) umfasst eine Steuervorrichtung (142) und mindestens ein von der Steuervorrichtung (142) getrennt ausgebildetes medizinisches Benutzerelement (140). Das medizinische Benutzerelement (140) und die Steuervorrichtung (142) sind eingerichtet, um drahtlos Daten auszutauschen. Das medizinische System (144) ist eingerichtet, um einen automatischen Zuordnungsschritt zu ermöglichen, wobei durch den automatischen Zuordnungsschritt ein Austausch persönlicher Daten zwischen dem medizinischen Benutzerelement (140) und der Steuervorrichtung (142) freigegeben wird. Das medizinische System (144) ist weiterhin eingerichtet, um den automatischen Zuordnungsschritt durch eine Zuordnungskopplung zwischen dem medizinischen Benutzerelement (140) und der Steuervorrichtung (142) automatisch zu initiieren. Das medizinische System (144) ist weiter eingerichtet, um nach dem Zuordnungsschritt eine Trennung der Zuordnungskopplung für einen medizinischen Betrieb des medizinischen Systems (144) zu ermöglichen.

## Beschreibung

### Gebiet der Erfindung

Die Erfindung betrifft ein medizinisches System mit einer Steuervorrichtung und mindestens einem von der Steuervorrichtung getrennt ausgebildeten medizinischen Benutzerelement, sowie ein Verfahren zum Betrieb eines medizinischen Systems. Derartige medizinische Systeme und Verfahren werden insbesondere zur Überwachung und/oder Steuerung mindestens einer Körperfunktion eines Benutzers eingesetzt, beispielsweise zur kontinuierlichen Überwachung einer Analytkonzentration in einer Körperflüssigkeit. Einsatzgebiete sind insbesondere im Krankenhausbereich, im Pflegebereich oder im Bereich der Home Care und/oder des Home Monitorings zu finden. Auch andere Einsatzgebiete sind jedoch grundsätzlich möglich.

### Stand der Technik

Aus der Medizintechnik sind zahlreiche Beispiele medizinischer Systeme bekannt, welche mehrere, voneinander getrennt ausgebildete Komponenten aufweisen, die miteinander kommunizieren sollen. Die vorliegende Erfindung betrifft insbesondere medizinische Systeme, welche mindestens ein medizinisches Benutzerelement umfassen. Unter einem Benutzerelement ist dabei, wie unten noch näher ausgeführt wird, ein persönliches medizinisches Element zu verstehen, beispielsweise mit einer Messfunktion und/oder einer Medikationsfunktion. Derartige Benutzerelemente werden in der Regel durch mindestens eine Steuervorrichtung angesteuert und/oder ausgelesen. Die Steuervorrichtung kann beispielsweise eine Benutzerschnittstelle bereitstellen, mittels derer ein Benutzer (beispielsweise ein Patient oder eine gesunde Person) das System steuern kann oder Informationen des Systems abfragen kann. Beispiele derartiger medizinischer Systeme sind in US 2005/0113886A1, in US 6,738,670B1 oder in US 6,752,155B2 aufgefiihrt.

Eine besondere Herausforderung bei derartigen medizinischen Systemen besteht in der Verbindung der einzelnen Komponenten dieser Systeme. Dabei ist grundsätzlich eine drahtlose Datenverbindung, also eine Verbindung, bei welcher nicht über künstliche Leitungen (wie beispielsweise Kabel, Schnittstellen oder Stecker) ein Datenaustausch erst ermöglicht wird, sondern der Datenaustausch über elektromagnetische Wellen erfolgt, einem drahtgebundenen Datenaustausch aus Praktikabilitätsgründen vorzuziehen. Bei einem drahtlosen Datenaustausch besteht jedoch in medizinischen Systemen die besondere Herausforderung darin, dass einerseits eine Interoperabilität der einzelnen Komponenten sichergestellt sein muss, ohne dass ein Benutzer (beispielsweise ein Patient in Kindesalter oder ältere Menschen) hierzu aufwendige technische Maßnahmen vornehmen muss. Andererseits muss jedoch gewährleistet sein, dass empfindliche persönliche Daten, wie beispielsweise Steuerbefehle, die an ein Benutzerelement übermittelt werden oder Messdaten, die von dem Benutzerelement an die Steuervorrichtung übermittelt werden, den korrekten Adressaten erreichen. So darf insbesondere die Operabilität, unter welcher beispielsweise eine Plug-and-Play-Interoperabilität verstanden werden kann, nicht derart weit geführt werden, dass automatisch neue Geräte in das medizinische System integriert werden, welche diesem nicht angehören sollen, wie beispielsweise Benutzerelemente fremder Benutzer. Unter Plug-and-Play-Konzepten werden dabei allgemein Konzepte verstanden, bei welchen zu Systemen neue Hardwarekomponenten hinzugefügt werden können und vom System erkannt werden, ohne dass hierfür ein Laden eines separaten Treibers erforderlich ist. Dies kann beispielsweise zu einer unerwünschten Preisgabe persönlicher Messdaten oder sogar zur fehlerhaften Adressierung von Steuerbefehlen führen, mit möglicherweise fatalen medizinischen Folgen. Zu diesem Zweck sind aus der Medizintechnik bereits Standards bekannt, welche diesem Zweck dienen und welche beispielsweise unter IEEE 1073 zusammengefasst sind.

Eine besondere Schwierigkeit bietet sich bei medizinischen Systemen, welche mindestens eine diagnostische Funktion aufweisen, beispielsweise in diagnostischen Messsystemen. Beispiele derartiger medizinischer Systeme sind so genannte kontinuierliche Glukose-überwachungssysteme (Conitnuous Monitoring Glucose Systems), welche derzeit bereits kommerziell erhältlich sind. Dabei werden bei gegenwärtigen Systemen die einzelnen Komponenten, wie beispielsweise Einweg-Komponenten (Disposables), wieder verwendbare Komponenten (Reusables) sowie Auslese- und Datenmanagement-Komponenten in der Regel zusammen in einem Set verkauft. Es ist jedoch auch das nachträgliche Erwerben und spätere in Betrieb Nehmen von Systemteilen möglich. Die einzelnen Komponenten werden dabei vom Benutzer einzeln in Betrieb genommen und gegebenenfalls aufeinander abgestimmt bzw. einander zugewiesen.

Insbesondere bei Diabetes-Self-Monitoring-Systemen, jedoch auch bei anderen diagnostischen Messsystemen, werden in der Regel Verbrauchsmaterialien eingesetzt, wie beispielsweise Sensoren. Diese Sensoren tragen teilweise komplexe, spezifische Informationen, die sie beim Einsatz dem Zielsystem, beispielsweise einer Steuervorrichtung, mitteilen müssen. Andererseits werden gesammelte Datensätze in der Regel zur weiterführenden Bearbeitung bzw. Verarbeitung an periphere Computersysteme gesendet. Derartige Aktivitäten bedürfen in der Regel eines hohen Maßes an Organisation und Koordination durch den Bediener.

Insbesondere in derartigen diagnostischen Messsystemen, jedoch auch in anders gestalteten medizinischen Systemen und vor allem bei neu aufkommenden Multi-Sensor-Systemen (beispielsweise körpernahen Netzwerken, Body Area Networks, BANs) ist die Entwicklung einer Plug-and-Play-Technologie jedoch bislang entweder noch gar nicht eingeführt oder nur unzureichend entwickelt. Dies führt in vielen Fällen dazu, dass Benutzer, die in der Regel nicht technisch ausgebildet sind, Schwierigkeiten beim Umgang mit der komplexen Technik haben, sofern die Benutzer nicht aufwendig eingewiesen und geschult werden. Eine derartige Einweisung und Schulung fordert jedoch vom System-Lieferanten hohe Anstrengungen bezüglich der Gestaltung von Benutzerhandbüchern oder Schulungsmaßnahmen.

Ein weiteres Problem besteht in vielen Fällen bei Verbrauchsmaterialien, wie beispielsweise Teststreifen oder anderen Arten von Sensoren. Hier müssen aus prozesstechnischen Gründen in vielen Fällen Kalibrationsdaten ermittelt und dem Verbrauchsmaterial in Form eines separaten Datenträgers mitgegeben werden. Diese Daten müssen bei der Inbetriebnahme des Verbrauchsmaterials dem Mess- bzw. Auswertegerät übertragen werden, so dass dieses gegebenenfalls eine Korrektur der ermittelten Messwerte durchführen kann. Daten und Verbrauchsmaterial sind jedoch in vielen Fällen physikalisch nicht zwangsweise einander zugeordnet, so dass das Verbrauchsmaterial und die Daten beim Neuintialisieren von Systemkomponenten, verstärkt jedoch auch beim Neubestücken mit Verbrauchsmaterial, vertauscht werden können. Dies kann zu falschen Messergebnissen führen, bis hin zu möglicherweise schwerwiegenden medizinischen Folgen.

Ferner können Probleme bei funktechnisch kommunizierenden Komponenten auftreten. Wie oben dargestellt, sind derartige Verbindungen zwischen den einzelnen Komponenten nicht eindeutig festgelegt und beispielsweise nicht eindeutig sichtbar, wie dies insbesondere bei durch Kabel oder optische Strecken verbundenen oder vernetzten Verbindungen möglich ist. Funkverbindungen sind beispielsweise durch oder über natürliche Hindernisse wie Wände oder Ähnliches hinweg möglich. Viele moderne Funknetze werden definitionsgemäß eigenmächtig aufgebaut, in dem sich potenzielle Netzwerkkomponenten selbständig identifizieren und einen möglichen Datenaustausch vorbereiten. Dies bedeutet, dass Komponenten der Netzwerke miteinander kommunizieren können, ohne dass der Benutzer hierfür aktiv eingreifen muss. Dabei werden jedoch in der Regel noch keine spezifischen Nutzerdaten, also insbesondere private Daten, ausgetauscht. Sollen derartige persönliche oder private Daten übertragen werden, so ist zuvor in der Regel eine so genannte Authentifizierung durchzuführen. Diese kann beispielsweise durch eine manuelle Eingabe von Nummern und/oder Codes direkt in die betreffenden Komponenten erfolgen, welche miteinander kommunizieren sollen. Problematisch an dieser Authentifizierung ist jedoch, dass diese eine aktive und komplexe Tätigkeit des Benutzers voraussetzt, welche insbesondere bei der Verwendung durch ältere Patienten oder Kinder, fehleranfällig sein kann. So können insbesondere bei der Eingabe langer Zahlenreihen, welche ohnehin mit einem beträchtlichen Aufwand verbunden ist, leicht Fehleingaben erfolgen, was wiederum zu Fehlfunktionen des Systems führen kann.

Diese Fehlfunktionen erhöhen das Risiko medizinischer Netzwerke, welche ohnehin beispielsweise aufgrund zahlreicher Möglichkeiten für eine Fehlbedienung mit einem Risiko behaftet sein können. So kann beispielsweise das Einsetzen von Verbrauchsmaterial und von Hilfsmaterialien, wie beispielsweise Batterien oder Speicherkomponenten durch einen Benutzer mit einer Fehlbedienung verbunden sein, beispielsweise einer Verpolung oder Fehlkontaktierungen. Diese kann zu Fehlfunktionen und Schäden im medizinischen Netzwerk führen. In der Regel ist somit für die Inbetriebnahme einzelner Systemkomponenten oder gesamter Netzwerke eine umfangreiche, oft schwer verständliche Dokumentation erforderlich, welche Kosten verursacht, Platz benötigt und in der Regel nicht oder erst im Fehlerfall vom Benutzer überhaupt wahrgenommen, bzw. gelesen wird.

### Aufgabe der Erfindung

Es ist daher eine Aufgabe der vorliegenden Erfindung, ein medizinisches System bereitzustellen, welches die Nachteile bekannter medizinischer Systeme zumindest weitgehend vermeidet. Insbesondere soll das System in der Lage sein, neue Systemkomponenten, insbesondere neue medizinische Verbrauchsmaterialien, eigenständig zu identifizieren und mit einem geringstmöglichen Benutzeraufwand sicher und zuverlässig zu integrieren.

### Offenbarung der Erfindung

Diese Aufgabe wird durch die Erfindung mit den Merkmalen der unabhängigen Ansprüche gelöst. Vorteilhafte Weiterbildungen der Erfindung, welche einzeln oder in Kombination realisierbar sind, sind in den abhängigen Patentansprüchen dargestellt. Dabei wird ein medizinisches System vorgeschlagen sowie ein Verfahren zum Betrieb eines medizinischen Systems, welches insbesondere auf ein erfindungsgemäßes medizinisches System angewandt werden kann. Dementsprechend kann für mögliche Ausgestaltungen des Verfahrens auf die beschriebenen Ausgestaltungen des medizinischen Systems verwiesen werden und umgekehrt.

Unter einem medizinischen System ist dabei allgemein ein System zu verstehen, welches mindestens zwei Komponenten umfasst, welche insbesondere räumlich getrennt voneinander ausgestaltet sein können und welches mindestens eine medizinische, insbesondere eine therapeutische und/oder diagnostische Funktion aufweist. Insbesondere kann es sich um ein System handeln, welches zur Überwachung und/oder Steuerung mindestens einer Körperfunktion eines Benutzers dient. Unter einer Körperfunktion ist dabei allgemein eine grundsätzlich beliebige Funktion und/oder Eigenschaft des Körpers des menschlichen oder tierischen Benutzers zu verstehen, beispielsweise eine physikalisch und/oder chemisch messbare Eigenschaft wie z.B. eine Analytkonzentration in einer Körperflüssigkeit und/oder eine Körperfunktion, welche mit einer Aktorik verbunden ist, beispielsweise eine Ventilfunktion, eine Muskelfunktion oder eine Ausschüttung bestimmter chemischer Substanzen. Verschiedene Beispiele werden unten näher aufgeführt. Bei dem Benutzer kann es sich grundsätzlich um einen Patienten handeln, jedoch auch grundsätzlich um gesunde Personen. Mindestens eine Komponente des medizinischen Systems, insbesondere das unten näher aufgeführte Benutzerelement, sollen dabei vorzugsweise von dem Benutzer, dessen Körperfunktion überwacht und/oder gesteuert werden soll, selbst getragen werden, vorzugsweise unmittelbar am oder im Körper.

Das medizinische System umfasst eine Steuervorrichtung. Besonders bevorzugt ist es, wenn das medizinische System genau eine derartige Steuervorrichtung umfasst, wobei jedoch auch medizinische Systeme denkbar sind, welche mehrere derartige Steuervorrichtungen umfassen, welche vorzugsweise ebenfalls kommunizieren können. Die Steuervorrichtung kann insbesondere tragbar ausgestaltet sein, so dass diese vom Benutzer selbst mitgeführt werden kann. Dementsprechend kann die Steuervorrichtung von ihren Abmessungen her dimensioniert sein, um beispielsweise in einer Tasche des Benutzers mitgeführt zu werden.

Unter einer Steuervorrichtung ist dabei grundsätzlich eine Vorrichtung zu verstehen, welche mindestens eine Benutzerschnittstelle aufweist, über welche ein Benutzer - vorzugsweise ohne Zuhilfenahme weiterer elektronischer Komponenten - Daten und/oder Befehle in das medizinische System eingeben kann und/oder Informationen aus dem medizinischen System auslesen kann. Beispielsweise kann die Steuervorrichtung zu diesem Zweck ein oder mehrere Bedienelemente umfassen, beispielsweise eine oder mehrere Tasten, ein oder mehrere Touchscreens, Spracheingaben, Trackballs, Schieber, Hebel, Wählscheiben, Scanner oder andere Arten von Bedienelementen oder Kombinationen der genannten oder andere Arten von Bedienelementen. Weiterhin kann die Steuervorrichtung, alternativ oder zusätzlich, ein oder mehrere Anzeigenelemente umfassen, beispielsweise um optisch und/oder akustisch und/oder haptisch (beispielsweise durch Vibrationen) Informationen an den Benutzer zu übermitteln. Beispielsweise kann mindestens ein Anzeigenelement in Form eines Displays umfasst sein, beispielsweise eines segmentierten Displays und/oder eines Matrix-Displays. Dieses kann auch kombiniert sein mit Bedienelementen, beispielsweise in Form eines Touch-Screens.

Die Steuervorrichtung kann beispielsweise, wie unten noch näher ausgeführt wird, ein einfaches Blutglukosemessgerät umfassen und/oder auch ein oder mehrere Geräte, welche aus nicht-medizinischen Bereichen stammen, wie beispielsweise Mobilfunkgeräte oder PDAs. Die Steuervorrichtung sollte somit eingerichtet sein, um einem Benutzer die Eingabe von Befehlen in das medizinische System, insbesondere zur Steuerung des unten noch näher ausgeführten medizinischen Benutzerelements, und/oder zum Auslesen von Informationen, beispielsweise Messdaten des medizinischen Benutzerelements, über die Steuervorrichtung zu ermöglichen.

Das medizinische System weist weiterhin mindestens ein von der Steuervorrichtung getrennt ausgebildetes medizinisches Benutzerelement auf. Unter einem medizinischen Benutzerelement ist grundsätzlich eine beliebige Vorrichtung mit einer medizinischen Funktion zu verstehen, welche vom Benutzer mitgeführt werden kann. Dieses Mitführen kann vorzugsweise unmittelbar am oder im Körper erfolgen. Die medizinische Funktion kann insbesondere eine therapeutische und/oder eine diagnostische Funktion aufweisen. Beispielsweise kann diese Funktion eine Messfunktion zur Erfassung bestimmter Daten aufweisen, insbesondere zur Erfassung von für den Benutzer charakteristische Daten, beispielsweise Daten, welche mit einer Körperfunktion zusammenhängen. Beispielsweise kann es sich dabei um Messdaten einer Analytkonzentration in einer Körperflüssigkeit des Benutzers handeln. Alternativ oder zusätzlich kann die medizinische Funktion eine Medikationsfunktion aufweisen, beispielsweise ein Verabreichung eines bestimmten Medikaments zu bestimmten Zeitpunkten und/oder in vorgegebenen Mengen bzw. Konzentrationen. Beispielsweise kann es sich dabei um die Funktion einer Insulinpumpe handeln. Wiederum alternativ oder zusätzlich kann die medizinische Funktion auch eine Aktorfunktion umfassen, beispielsweise eine Schrittmacherfunktion und/oder eine Ventilfunktion. Verschiedene Beispiele werden unten näher ausgeführt. Das medizinische Benutzerelement kann mindestens ein Verbrauchselement umfassen, da die im Folgenden beschriebenen Vorteile des medizinischen Systems sich insbesondere beim Austausch von Verbrauchsmitteln positiv bemerkbar machen.

Das medizinische Benutzerelement und die Steuervorrichtung sind eingerichtet, um drahtlos Daten auszutauschen. Unter Daten können dabei allgemein Informationen und/oder Befehle subsumiert werden, beispielsweise Messdaten und/oder Steuerbefehle. Unter einem drahtlosen Datenaustausch ist dabei ein Datenaustausch zu verstehen, bei welchem nicht durch eine spezielle Hardware, wie beispielsweise ein Kabel, eine Datenverarbeitung durch den Benutzer eingerichtet werden muss. Der drahtlose Datenaustausch soll vielmehr vorzugsweise über elektromagnetische Wellen und/oder konduktive Leitmechanismen stattfinden, welche direkt oder auch beispielsweise über ein Körpernetzwerk, welches Körperteile des Benutzers als leitende Elemente einschließt, übertragen werden. Vorzugsweise umfasst der drahtlose Datenaustausch einen Datenaustausch über Funk, vorzugsweise einen Datenaustausch, bei welchem die Steuervorrichtung und das medizinische Benutzerelement nicht in einer bestimmten Orientierung zueinander angeordnet sein müssen, wie beispielsweise bei einer infraroten Datenverbindung. Grundsätzlich sind jedoch auch andere Arten von drahtlosem Datenaustausch implementierbar, beispielsweise Leitwerte am/im Körper oder kapazitive, dielektrische Verschiebeströme, beispielsweise in Form so genannter körpernaher Netzwerke (Body Area Network, BAN). Besonders bevorzugt ist Datenaustausch über eine Funkverbindung, vorzugsweise eine Fernfeld-Funkverbindung.

Das medizinische System ist eingerichtet, um einen automatischen Zuordnungsschritt zu ermöglichen, durch welchen ein Austausch persönlicher Daten zwischen dem medizinischen Benutzerelement und der Steuervorrichtung freigegeben wird. Unter automatisch ist in diesem Zusammenhang insbesondere zu verstehen, dass der Zuordnungsschritt ohne komplexe Benutzerhandlungen erfolgen kann, beispielsweise ohne Eingabe komplexer alphanumerischer Codes, ohne Austausch von Speicherchips oder ähnlichen Datenträgern, durch den Benutzer. Der Zuordnungsschritt ist somit ein Verfahrensschritt, durch welchen eine Kommunikation zwischen der Steuervorrichtung und dem medizinischen Benutzerelement ermöglicht wird, bei welcher auch sensitive, persönliche Daten ausgetauscht werden können. Bereits vor der Durchführung des mindestens einen Zuordnungsschritts können das medizinische Benutzerelement und die Steuervorrichtung kommunizieren, beispielsweise in dem ein einfaches Erkennungssignal übermittelt wird, welches die reine Anwesenheit des Benutzerelements in der Nähe der Steuervorrichtung signalisiert oder umgekehrt. Auf diese Weise beispielsweise vor dem Zuordnungsschritt eine vorläufige Kommunikation erfolgen, mittels derer beispielsweise allgemeine Daten ausgetauscht werden können.

Unter persönlichen Daten, deren Austausch erst nach erfolgreicher Durchführung des Zuordnungsschritts möglich sein soll, sind allgemein Daten und/oder Steuerbefehle zu verstehen, welche für die Funktionalität des medizinischen Systems wesentlich sind, beispielsweise für eine therapeutische und/oder diagnostische Funktion eines bestimmten medizinischen Benutzerelements. Dabei kann es sich beispielsweise um Messdaten handeln, die an diesem bestimmten Benutzer, der das medizinische System und/oder das bestimmte medizinische Benutzerelement verwendet, erfasst wurden, handeln und/oder um Steuerbefehle, welche für ein spezielles medizinisches Benutzerelement bestimmt sind und welche vorzugsweise nicht an andere medizinische Benutzerelemente übermittelt werden sollen. Die Klassifikation von Daten als persönliche Daten kann beispielsweise in einer oder mehreren Komponenten des medizinischen Systems festgelegt werden, beispielsweise in der Steuervorrichtung und/oder in dem medizinischen Benutzerelement. Beispielsweise kann die Steuervorrichtung derart eingerichtet sein, dass in dieser bestimmte Steuerbefehle als persönliche Daten eingestuft werden, welche erst nach erfolgreicher Durchführung des Zuordnungsschritts an ein bestimmtes Benutzerelement, mit welchem der Zuordnungsschritt durchgeführt wurde, übermittelt werden dürfen. Umgekehrt können beispielsweise bestimmte Messdaten oder Arten von Messdaten in einem medizinischen Benutzerelement als persönliche Daten klassifiziert werden, so dass eine Übermittlung dieser Messdaten an die Steuervorrichtung beispielsweise erst dann erfolgen kann, wenn mit dieser bestimmten Steuervorrichtung ein Zuordnungsschritt erfolgreich beendet wurde.

Der Austausch der persönlichen Daten zwischen dem medizinischen Benutzerelement und der Steuervorrichtung kann unidirektional in einer der beiden Richtungen, also beispielsweise von der Steuervorrichtung zum medizinischen Benutzerelement oder umgekehrt, oder bidirektional erfolgen. So können beispielsweise Messdaten von dem medizinischen Benutzerelement zur Steuervorrichtung übermittelt werden und Steuerbefehle von der Steuervorrichtung zum medizinischen Benutzerelement. Auch andere Ausgestaltungen sind jedoch grundsätzlich möglich.

Das medizinische System ist erfindungsgemäß eingerichtet, um den automatischen Zuordnungsschritt durch eine Zuordnungskopplung zwischen dem medizinischen Benutzerelement und der Steuervorrichtung automatisch zu initiieren. Dies bedeutet, dass der Zuordnungsschritt erfolgreich ist, wenn das medizinische System, vorzugsweise sowohl die Steuervorrichtung als auch das medizinische Benutzerelement, erkennt, dass die Zuordnungskopplung hergestellt worden ist. Unter einer Zuordnungskopplung ist dabei, wie unten noch näher ausgeführt wird, eine vorgegebene Handhabung der Steuervorrichtung und des medizinischen Benutzerelements durch den Benutzer selbst und/oder eine andere Person zu verstehen, welche sich von einer Handhabung im normalen Betrieb, beispielsweise einem medizinischen Betrieb, in welchem das medizinische System regulär arbeitet und in welchem die persönlichen Daten ausgetauscht werden, unterscheidet. In anderen Worten soll die Zuordnungskopplung eine bewusste Handhabung des medizinischen Benutzerelements und der Steuervorrichtung durch den Benutzer und/oder eine andere Person beinhalten, welche im medizinischen Betrieb nicht oder nur mit sehr geringer Wahrscheinlichkeit zufällig auftritt und durch welche der Zuordnungsschritt initiiert wird. Diese Zuordnungskopplung soll vorzugsweise keinerlei komplexe Handhabung, wie beispielsweise den Austausch von Speicherelementen oder die Eingabe alphanumerischer Zeichen beinhalten.

Das medizinische System ist weiterhin eingerichtet, um nach dem Zuordnungsschritt, also nach erfolgreicher Durchführung des Zuordnungsschritts, eine Trennung der Zuordnungskopplung für den medizinischen Betrieb des medizinischen Systems zu ermöglichen. Dies bedeutet, dass die Zuordnungskopplung lediglich temporär hergestellt wird, wobei diese anschließend für den medizinischen Betrieb getrennt werden kann. Im medizinischen Betrieb, in welchem die persönlichen Daten ausgetauscht werden, besteht somit die Zuordnungskopplung nicht mehr, sondern es findet vorzugsweise ausschließlich der Austausch der persönlichen Daten über die drahtlose Datenverbindung statt, wobei jedoch beispielsweise eine während des Bestehens der Zuordnungskopplung ausgetauschte Zuordnungsidentifikation weiter verwendet werden kann. Unter einem medizinischen Betrieb ist dabei der Normalbetrieb des medizinischen Systems zu verstehen, beispielsweise ein diagnostischer Betrieb und/oder ein therapeutischer Betrieb, beispielsweise ein Messbetrieb. In diesem können die medizinischen Daten ausgetauscht werden.

Das vorgeschlagene medizinische System ermöglicht somit auf einfache Weise einen Plug-and-Play-Betrieb, in welchem auch auf einfache Weise neue medizinische Benutzerelemente in das medizinische System integriert oder aus diesem medizinischen System ausgetauscht werden können. Durch die Zuordnungskopplung, welche den Zuordnungsschritt initiiert, ist auf einfache und sichere Weise eine Initiierung des Austauschs persönlicher Daten möglich, ohne dass eine komplexe Handhabung durch den Benutzer erforderlich ist, wie beispielsweise die Eingabe alphanumerischer Codes und/oder den Austausch von Speicherelementen. Derartige Handlungen können jedoch zusätzlich vorgesehen sein.

Das medizinische System ermöglicht somit die Kommunikation zwischen der Steuervorrichtung und dem medizinischen Benutzerelement, wobei zunächst die beiden Komponenten in dem automatischen Zuordnungsschritt auf spezielle Art und Weise miteinander in Verbindung treten, vorzugsweise ohne dass der Benutzer hierbei Daten eingeben oder einen Code austauschen muss bzw. Tasten drücken muss. Eine Authentifizierung kann automatisch zwischen der Steuervorrichtung und dem medizinischen Benutzerelement stattfinden, so dass durch den Zuordnungsschritt durch die bewusst vom Benutzer herbeigeführte Zuordnungskopplung eine Paarbildung (Pairing) zwischen der Steuervorrichtung und dem medizinischen Benutzerelement herbeigeführt werden kann. Die Authentifizierung, welche den anschließenden Austausch persönlicher Daten ermöglicht, kann automatisch zwischen den Komponenten stattfinden, so dass anschließend ein Datenaustausch ohne weiteren Pairing-Vorgang stattfinden kann, im Gegensatz zu bekannten medizinischen Systemen. So kann beispielsweise bei einer Inbetriebnahme eines medizinischen Benutzerelements, beispielsweise bei einer Neuinbetriebnahme eines Sensors, ein automatischer Zuordnungsschritt in Form eines automatischen Pairings erfolgen, welcher es ermöglicht, das medizinische Benutzerelement und/oder den Benutzer selbst zu authentifizieren.

Bevorzugte Ausführungsformen des medizinischen Systems betreffen insbesondere die Ausgestaltung der Zuordnungskopplung. Diese Zuordnungskopplung, welche beispielsweise ein automatisches Pairing ermöglicht, kann, wie oben aufgeführt, grundsätzlich eine bewusste Handlung des Benutzers und/oder einer anderen Person beinhalten, welche zur Herbeiführung der Zuordnungskopplung und/oder zum Auslösen des Zuordnungsschritts erforderlich ist. Im Rahmen der vorliegenden Erfindung wird bezüglich der Vornahme dieser Handlung nicht zwischen dem Fall unterschieden, dass die Behandlung durch den Benutzer selbst vorgenommen wird oder durch eine andere Person, beispielsweise einen Arzt, Apotheker oder Medizintechniker. Diese Möglichkeiten sollen vom Begriff der Vornahme der Handlung durch den Benutzer mit umfasst sein. Die bewusste Handlung soll insbesondere, wie oben aufgeführt, einfach und wenig fehleranfällig ausgestaltet sein und soll sich von anderen Handlungen, welche im medizinischen Betrieb des medizinischen Systems auftreten oder welche unbewusst durchgeführt werden könnten, unterscheiden. Insbesondere sollte keine Eingabe alphanumerischer Zeichen durch den Benutzer und/oder einen Austausch von Speicherelementen und/oder eine Eingabe von Daten und/oder Codes und/oder ein Betätigen von Tasten erforderlich sein.

In einer ersten möglichen Ausgestaltung kann die Zuordnungskopplung eine vorübergehende Kopplungsausrichtung zwischen dem medizinischen Benutzerelement und der Steuervorrichtung umfassen, wobei die Kopplungsausrichtung von einer Ausrichtung im medizinischen Betrieb und vorzugsweise auch von einer zufälligen Ausrichtung der Komponenten des medizinischen Systems verschieden ist. Diese Kopplungsausrichtung kann beispielsweise eine bestimmte Positionierung und/oder räumliche Orientierung der Steuervorrichtung und des medizinischen Benutzerelements zueinander beinhalten. Beispielsweise kann diese Kopplungsausrichtung eine bestimmte räumliche Nähe, beispielsweise ein Unterschreiten eines Mindestabstands, bis hin zu einem (vorzugsweise nicht-zufälligen) physikalischen Kontakt zwischen dem medizinischen Benutzerelement und der Steuervorrichtung umfassen. Diese Erzielung einer räumlichen Nähe zur Herbeiführung der Kopplungsausrichtung kann auch als "Pairing By Near Closing" bezeichnet werden. Vorzugsweise ist diese Kopplungsausrichtung derart ausgestaltet, dass diese bei einer normalen Handhabung des medizinischen Systems nicht auftritt, so dass vorzugsweise keine zufällige, ungewollte Herbeiführung der Kopplungsausrichtung möglich ist. Beispiele einer derartigen Kopplungsausrichtung werden unten aufgeführt.

Alternativ oder zusätzlich kann die Zuordnungskopplung auch eine vorübergehende physikalische Kopplungsverbindung zwischen dem medizinischen Benutzerelement und der Steuervorrichtung umfassen, welche von dem Benutzer gezielt herbeigeführt werden kann und anschließend wieder getrennt werden kann. Diese vorübergehende physikalische Kopplungsverbindung kann beispielsweise über eine Kopplungsschnittstelle, eine Dockingstation, eine drahtgebundene Verbindung, eine drahtlose Nahfeldverbindung oder eine Infrarotverbindung erfolgen. Im Gegensatz zur drahtlosen Verbindung zum Austausch der persönlichen Daten im medizinischen Betrieb, welche vorzugsweise nicht-galvanisch erfolgt, kann bei dieser vorübergehenden physikalischen Kopplungsverbindung auch eine vorübergehende galvanische Kopplung erfolgen, beispielsweise über ein Kopplungskabel oder die genannte Dockingstation. So können beispielsweise das Benutzerelement und die Steuervorrichtung nacheinander oder gleichzeitig in eine Dockingstation eingesteckt werden, so dass durch diese Handhabung die Kopplungsverbindung hergestellt wird und der Zuordnungsschritt initiiert wird. Das medizinische Benutzerelement und die Steuervorrichtung können eindeutig einander zugeordnet werden.

Unter einer Dockingstation ist dabei grundsätzlich eine stationäre Vorrichtung zu verstehen, welche beispielsweise über eine stationäre Stromversorgung verfügen kann und/oder auch über eine eigene, portable Stromversorgung, welche beispielsweise einen Austausch von Authentifizierungsinformationen zwischen dem medizinischen Benutzerelement und der Steuervorrichtung und/oder ein Austausch anderer Art von Zuordnungsidentifikationen ermöglicht. Auch eine portable Ausgestaltung der Dockingstation ist grundsätzlich möglich. Auch Einrichtungen zum Transport und/oder zur Energieversorgung, beispielsweise in Form eines Ladeteils, des medizinischen Benutzerelements und/oder der Steuervorrichtung können ganz oder teilweise als Dockingstation ausgebildet sein und/oder in einer Dockingstation enthalten sein.

Alternativ oder zusätzlich zu der Dockingstation kann auch eine vorübergehende drahtgebundene Verbindung hergestellt werden, beispielsweise indem ein Benutzer das medizinische Benutzerelement und die Steuervorrichtung vorübergehend mittels eines Kabels verbindet. Eine Infrarotverbindung kann beispielsweise eine bewusste Ausrichtung des medizinischen Benutzerelements relativ zur Steuervorrichtung durch den Benutzer beinhalten, so dass jeweils Infrarotschnittstellen dieser Elemente miteinander kommunizieren können. Anschließend kann diese Infrarotverbindung wieder getrennt werden, beispielsweise indem das medizinische Benutzerelement und die Steuervorrichtung derart positioniert werden, dass eine Infrarotkommunikation nicht mehr erfolgen kann.

Wiederum alternativ oder zusätzlich kann die drahtlose Nahfeldverbindung beispielsweise eine Verbindung über eine Hochfrequenzschnittstelle beinhalten, welche lediglich einen Datenaustausch über kurze Distanzen ermöglicht, beispielsweise Distanzen von einem Mindestabstand oder weniger, beispielsweise wenigen 10 cm. Beispielsweise können typischerweise in der RFID-Technologie verwendete Übertragungstechniken verwendet werden, welche anschließend für den Austausch von persönlichen Daten jedoch nicht geeignet sind. Beispielsweise kann das medizinische Benutzerelement einen RFID-Chip und/oder einen Transponder umfassen, welcher lediglich in einer bestimmten Kopplungsausrichtung zwischen dem medizinischen Benutzerelement und der Steuervorrichtung durch ein Lesegerät der Steuervorrichtung auslesbar ist, wobei die RFID-Verbindung vorzugsweise anschließend wieder getrennt werden kann. Der anschließende Austausch persönlicher Daten im medizinischen Betrieb erfolgt dann nicht mehr über die genannte RFID-Verbindung bzw. Nahfeldverbindung, sondern über die oben beschriebene drahtlose Verbindung. Bei dieser Ausgestaltung oder auch bei anderen Ausgestaltungen sollten somit sowohl das medizinische Benutzerelement als auch die Steuervorrichtung über mindestens zwei Schnittstellen verfügen, eine Schnittstelle für die Durchführung des automatischen Zuordnungsschritts und eine Schnittstelle für den drahtlosen Datenaustausch zumindest der persönlichen Daten während des medizinischen Betriebs.

Alternativ oder zusätzlich zu den genannten Möglichkeiten kann die Zuordnungskopplung auch eine indirekte Zuordnungskopplung über mindestens ein Koppelelement umfassen. Dabei soll die Zuordnungskopplung derart ausgestaltet sein, dass das medizinische Benutzerelement und die Steuervorrichtung gleichzeitig oder nacheinander vorübergehend mit dem Koppelelement verbindbar sind, um den Zuordnungsschritt zu initiieren. Unter einem Koppelelement kann dabei grundsätzlich ein beliebiger Gegenstand verstanden werden, mit dem das medizinische Benutzerelement und die Steuervorrichtung gleichzeitig oder nacheinander verbindbar sind, insbesondere zum Austausch einer mehrerer Zuordnungsidentifikationen. Auf diese Weise kann die Zuordnung in dem Zuordnungsschritt, beispielsweise eine Authentifizierung, mittelbar über das Koppelelement erfolgen. Das Koppelelement kann beispielsweise die oben beschriebene vorübergehende physikalische Kopplungsverbindung beinhalten und/oder bereitstellen. Das Koppelelement kann beispielsweise ein lebloser Gegenstand sein, kann jedoch auch beispielsweise einen oder mehrere Körperteile des Benutzers umfassen, über welchen beispielsweise Signale ausgetauscht werden können. Auch biometrische Kenndaten in Form biometrischer Merkmale können, wie unten noch näher ausgeführt wird, über ein Körperteil des Benutzers ausgetauscht werden bzw. von dem Körperteil des Benutzers gleichzeitig oder nacheinander sowohl auf das medizinische Benutzerelement als auch auf die Steuervorrichtung übertragen werden, so dass das medizinische Benutzerelement und die Steuervorrichtung einander zugeordnet werden können.

Das medizinische System kann eingerichtet sein, um über die Zuordnungskopplung (beispielsweise das oben genannte Koppelelement) mindestens eine Zuordnungsidentifikation auszutauschen. Dieser Austausch kann beispielsweise unidirektional von der Steuervorrichtung zu dem medizinischen Benutzerelement oder umgekehrt erfolgen oder bidirektional. Alternativ oder zusätzlich kann der Austausch auch beispielsweise ein drittes Element, wie beispielsweise das oben genannte Koppelelement, umfassen, welches die Zuordnungsidentifikation bereitstellt. In diesem Fall verfügen sowohl die Steuervorrichtung als auch das medizinische Benutzerelement über die korrespondierende bzw. identische Zuordnungsidentifikation, so dass eine eindeutige Zuordnung dieser beiden Komponenten zueinander erfolgen kann.

Auf diese Weise kann beispielsweise später ein einziger Datenmanager in Form der Steuervorrichtung bereitgestellt werden, der auf gezielte Art und Weise mit jeglichen zusätzlichen medizinischen Benutzerelementen, beispielsweise Messvorrichtungen, die der Patient benutzt, derart kommunizieren kann, dass der Datenmanager zunächst im Zuordnungsschritt Daten in Form der Zuordnungsidentifikation übermittelt erhält, die ihn dazu befähigen, in späteren normalen Datenaustauschprozessen während des medizinischen Betriebs zu erkennen, dass diese Daten von dem bestimmten medizinischen Benutzerelement bzw. von dem bestimmten Benutzer dieses medizinischen Benutzerelements stammen. Auf diese Weise kann ein und derselbe Datenmanager auch mit mehreren medizinischen Benutzerelementen in Wechselwirkung treten, wobei stets eine eindeutige Zuordnung der ausgetauschten Daten, beispielsweise der persönlichen Daten, zu einem bestimmten medizinischen Benutzerelement erfolgen kann. Hierdurch wird die Handhabungssicherheit des medizinischen Systems stark erhöht, da zum einen die Steuervorrichtung Daten verschiedener medizinischer Benutzerelemente unterscheiden kann und da zum anderen durch den genannten Zuordnungsschritt ein versehentliches Einbinden fremder medizinischer Benutzerelemente, beispielsweise dritter Benutzer, in das medizinische System, beispielsweise ein Netzwerk des medizinischen Systems, vermieden kann. Die Steuervorrichtung kann eingerichtet sein, um in diesem medizinischen Betrieb anhand der Zuordnungsidentifikation von dem medizinischen Benutzerelement übermittelte Daten, insbesondere persönliche Daten, diesem medizinischen Benutzerelement zuzuordnen und beispielsweise entsprechende weitere Schritte vorzunehmen, wie beispielsweise eine Datenauswertung, eine Speicherung der Daten oder Ähnliches. Allerdings könnte auch eine gezielte Einbindung dritter Personen, welche bewusst autorisiert sind, in ein oder mehrere medizinische Systeme erfolgen, _ oder mehrere medizinische Systeme könnten teilweise bauteilgleich sein. So könnte beispielsweise eine Steuervorrichtung auch Bestandteil mehrerer medizinischer Systeme sein, die grundsätzlich getrennt verwendet werden können. So könnten beispielsweise mehrere Benutzer, beispielsweise ein Ehepaar, eine gemeinsame Steuervorrichtung verwenden, beispielsweise einen gemeinsamen Datenmanager. Dieser kann dann beispielsweise in einem ersten medizinischen System eines ersten Benutzers und in einem zweiten medizinischen System eines zweiten Benutzers eingesetzt werden. Das erste medizinische System und das zweite medizinische System können unterschiedliche medizinischer Benutzerelemente umfassen. Über die jeweilige Zuordnungsidentifikation kann dann der aktuelle Betrieb einem bestimmten medizinischen System zugeordnet werden, so dass beispielsweise zu jedem Zeitpunkt, zu welchem persönliche Daten ausgetauscht werden, für die Steuervorrichtung erkennbar ist, ob diese persönlichen Daten dem ersten medizinischen System oder dem zweiten medizinischen System zuzuordnen sind. Entsprechend kann beispielsweise eine Auswertung und/oder Verarbeitung der persönlichen Daten getrennt erfolgen, so dass beispielsweise die unterschiedlichen Benutzer jeweils nur auf ihre persönlichen Daten zugreifen können und/oder dass eine Verwechslung und/oder Vermischung der persönlichen Daten ausgeschlossen ist.

Eine weitere vorteilhafte Ausgestaltung des medizinischen Systems betrifft die bereits oben erwähnte Zuordnungskopplung, durch welche der Zuordnungsschritt ermöglicht wird. Wie oben dargestellt, kann die Zuordnungskopplung beispielsweise eine vorübergehende, vorgegebene Kopplungsausrichtung zwischen dem medizinischen Benutzerelement und der Steuervorrichtung umfassen. Besonders bevorzugt ist es, wenn diese Kopplungsausrichtung durch eine äußere Gestaltung des medizinischen Systems oder einzelner Komponenten des medizinischen Systems unterstützt wird, so dass beispielsweise ein Benutzer eindeutig erkennt, wie die Kopplungsausrichtung zu erfolgen hat. Auf diese Weise kann durch eine äußere Formgebung des medizinischen Systems ein Bedienungsfehler bei der Herbeiführung der Kopplungsausrichtung vermieden werden. Dementsprechend ist es besonders bevorzugt, wenn das medizinische Benutzerelement und die Steuervorrichtung in ihrer äußeren Gestalt eingerichtet sind, um eine definierte vorübergehende Kopplungsausrichtung zwischen dem medizinischen Benutzerelement und der Steuervorrichtung zu ermöglichen. Besonders bevorzugt ist es, wenn die Steuervorrichtung ein Gehäuse aufweist, welches mindestens eine einer äußeren Gestalt des medizinischen Benutzerelements entsprechende Vertiefung aufweist. Dabei soll das medizinische Benutzerelement zur Erzeugung der Zuordnungskopplung vorübergehend in die Vertiefung einbringbar sein. Alternativ oder zusätzlich kann das Gehäuse auch erhaben ausgestaltet sein, also mindestens eine Erhöhung aufweisen, welche ebenfalls der äußeren Gestalt des medizinischen Benutzerelements entsprechen kann, wobei beispielsweise das medizinische Benutzerelement zur Erzeugung der Zuordnungskopplung auf die Erhöhung aufbringbar ist. Das medizinische Benutzerelement kann dementsprechend eine Tasche und/oder Vertiefung aufweisen, die der Erhöhung entspricht. Allgemein können, in einer weitesten Begriffsdefinition der Begriffe "Vertiefung" und "Erhöhung", das medizinische Benutzerelement und die Steuervorrichtung zueinander korrespondierende männliche und weibliche Verbindungselemente aufweisen, welche zur Herstellung der Zuordnungskopplung verwendet werden können. Auf diese Weise können die Steuervorrichtung und das medizinische Benutzerelement beispielsweise ganz oder teilweise in ihrer äußeren Form als "Schloss" und "Schlüssel" (oder umgekehrt) ausgestaltet sein, um eine eindeutige Zuordnungskopplung zu ermöglichen, welche vorzugsweise nicht versehentlich herbeigeführt werden kann.

Die Vertiefung kann beispielsweise eine Tasche, einen Eingabeschlitz, eine Ausbuchtung, eine Nut oder Kombinationen der genannten und/oder anderer Vertiefungen umfassen. Besonders bevorzugt ist es, wenn die Vertiefung von ihrer äußeren Gestalt her visuell für den Benutzer eindeutig der Gestalt des medizinischen Benutzerelements zuzuordnen ist, so dass intuitiv vom Benutzer erkannt wird, dass das medizinische Benutzerelement vollständig oder teilweise in diese Vertiefung eingefügt werden muss, um die Zuordnungskopplung herbeizuführen und damit den automatischen Zuordnungsschritt automatisch, d.h. vorzugsweise ohne Vornahme weiterer Handlungen, zu initiieren. Auf diese Weise können Fehlbedienungen weiter reduziert oder vorzugsweise vollständig vermieden werden.

Auf diese oder auf andere Weise lässt sich das oben bereits beschriebene "Pairing By Near Closing" bewerkstelligen, durch welches die vorübergehende vorgegebene Kopplungsausrichtung herbeigeführt wird. Auf diese Weise können beispielsweise die Steuervorrichtung und das medizinische Benutzerelement auf einen Authentifizierungsvorgang während des Zuordnungsschritts vorbereitet werden.

Eine weitere, ebenfalls alternativ oder zusätzlich einsetzbare Möglichkeit der Ausgestaltung des medizinischen Systems bzw. der Zuordnungskopplung wurde oben bereits angesprochen. Hierbei umfasst die Zuordnungskopplung einen Austausch biometrischer Merkmale. Dementsprechend können die Steuervorrichtung und das medizinische Benutzerelement beispielsweise zum Zweck der Zuordnungskopplung jeweils eine Schnittstelle in Form einer oder mehrerer Erfassungsvorrichtungen zur Erfassung eines oder mehrerer biometrischer Merkmale des Benutzers aufweisen. Alternativ oder zusätzlich zum Benutzer selbst kann insbesondere auch in diesem Fall auch eine dritte Person die biometrischen Merkmale bereitstellen, um die Zuordnung zu erzielen, beispielsweise ein Arzt, ein Apotheker, einen Pfleger, ein Ehepartner oder ein Medizintechniker, welcher das medizinische System für den Benutzer einrichtet.

Unter biometrischen Merkmalen sind dabei allgemein eindeutige biometrische Informationen zu verstehen, welche als Zuordnungsidentifikation dienen können. Beispiele derartiger biometrischer Merkmale sind eine Gestaltung einer Iris des Benutzers, Fingerabdrücke oder Ähnliches. Zu diesem Zweck kann die Erfassungsvorrichtung beispielsweise einen Fingerabdruckscanner, einen Irisscanner oder ähnliche Erfassungsvorrichtungen umfassen, so dass das selbe biometrische Merkmal einerseits von dem medizinischen Benutzerelement und andererseits von der Steuervorrichtung erfasst werden kann, um diese eindeutig einander zuzuordnen. Die Zuordnungskopplung kann dann eine Handhabung des medizinischen Benutzerelements und der Steuervorrichtung durch den Benutzer (oder sinngemäß eine dritte Person) umfassen, welche gleichzeitig oder nacheinander erfolgen kann, wobei das medizinische System eingerichtet ist, um bei Erkennung desselben biometrischen Merkmals durch die Erfassungsvorrichtung den Zuordnungsschritt zu initiieren. Das erfasste biometrische Merkmal dient somit als Zuordnungsidentifikation im Sinne der obigen Beschreibung oder zumindest als Teil dieser Zuordnungsidentifikation. Die Handhabung durch den Benutzer erfolgt beispielsweise mittels eines Körperteils, beispielsweise einer Hand, wobei beispielsweise die biometrischen Merkmale erfasst werden können, insbesondere ein Fingerabdruck der Hand. Die Erfassungsvorrichtungen können dabei vorzugsweise derart ausgestaltet sein, dass das biometrische Merkmal im normalen Betrieb, beispielsweise dem normalen medizinischen Betrieb, nicht erfasst wird, beispielsweise durch eine entsprechende Abdeckung und/oder eine Anordnung an Stellen, welche üblicherweise nicht mit dem betreffenden Körperteil in Berührung kommen.

Das medizinische System kann insbesondere eingerichtet sein, um bei oder nach erfolgreicher Vollendung des Zuordnungsschritts automatisch den medizinischen Betrieb ganz oder teilweise zu starten. Beispielsweise kann automatisch ein Messbetrieb eines Sensors gestartet werden. Eine weitere Handhabung durch einen Benutzer ist in diesem Fall nicht erforderlich, sondern es entsteht ein echtes Plug-and-Play-System, welches nach dem Zuordnungsschritt automatisch seinen Betrieb aufnehmen kann und beispielsweise persönliche Daten austauschen kann.

Das medizinische System kann ein oder mehrere medizinische Benutzerelemente umfassen. Sind mehrere medizinische Benutzerelemente vorgesehen, so können die Steuervorrichtung und die medizinischen Benutzerelemente ein medizinisches Netzwerk bilden. Dieses medizinische Netzwerk kann darüber hinaus weitere Komponenten umfassen, welche nicht unter den Begriff des medizinischen Benutzerelements oder unter den Begriff der Steuervorrichtung fallen, wie beispielsweise Vorrichtungen, welche keine medizinische Funktion aufweisen. So können beispielsweise ein Schnittstellengerät und/oder ein Speichergerät vorgesehen sein, um Daten zwischen dem medizinischen Netzwerk und anderen Komponenten auszutauschen, beispielsweise mit anderen Netzen. Die Anzahl zuzuordnender Komponenten und/oder die zeitliche Zuordnung und Reihenfolge sind grundsätzlich nicht begrenzt bzw. vorgeschrieben. So kann beispielsweise optional ein Datenloggermodul vorgesehen sein, beispielsweise für Evaluierungszwecke der persönlichen Daten und/oder anderer Daten. Alternativ oder zusätzlich kann der Benutzer beispielsweise von dem medizinischen Benutzerelement, beispielsweise einem Sensor gesammelten persönlichen Daten direkt in einem PC-System, beispielsweise bei einem Arzt, auswerten, beispielsweise indem diese auf das PC-System geladen werden (Upload). Auch dieser Vorgang kann ein Pairing erfordern, beispielsweise unter Vornahme eines Zuordnungsschritts gemäß der obigen Beschreibung. Das medizinische System kann weiterhin eingerichtet sein, um mittels weiterer automatischer Zuordnungsschritte im Sinne der obigen Beschreibung eine automatische Aufnahme weiterer medizinischer Benutzerelemente zu ermöglichen. Auf diese Weise lässt sich das medizinische Netzwerk nach dem Plug-and-Play-Prinzip erweitern, wobei jedoch stets sichergestellt sein kann, dass der Austausch persönlicher Daten lediglich zwischen einander zugeordneten Elementen, beispielsweise einer bestimmten Steuervorrichtung und einem bestimmten medizinischen Benutzerelement, erfolgt.

Wie oben beschrieben, kann die Steuervorrichtung beispielsweise mindestens eine Benutzerschnittstelle zum Austausch von Daten und/oder Befehlen mit dem Benutzer umfassen, insbesondere ein Anzeigenelement und/oder ein Bedienelement. Vorzugsweise umfasst das medizinische Benutzerelement hingegen keine derartigen Elemente, welche einen direkten Zugriff des Benutzers auf Funktionen und/oder Daten des medizinischen Bedienelements ermöglichen, so dass das medizinische Benutzerelement beispielsweise ganz oder teilweise als kostengünstiges, einfach gestaltetes Einweg-Element (Disposable) ausgestaltet sein kann. Insbesondere kann das medizinische Benutzerelement auch ohne Bedienelemente und/oder Anzeigenelemente ausgestaltet werden, so dass eine Bedienung des medizinischen Systems beispielsweise vollständig über die Steuervorrichtung erfolgen kann. Die Steuervorrichtung kann insbesondere mindestens eine Datenverarbeitungsvorrichtung umfassen, beispielsweise einen Mikrocomputer. Die Datenverarbeitungsvorrichtung kann eingerichtet sein, um die persönlichen Daten zumindest teilweise zu verarbeiten. Unter einer Verarbeitung in diesem Sinne sind allgemein eine Speicherung der Daten, eine Darstellung der Daten für einen Benutzer, eine Anzeige von Trends oder sonstigen Messergebnissen, eine zumindest teilweise Analyse der Daten (beispielsweise durch eine Filterung, Auswertung oder Ähnliches) oder Datenbankfunktionen oder Kombinationen der genannten oder anderer Verarbeitungsfunktionen zu verstehen.

Die Steuervorrichtung kann, wie oben beschrieben, beispielsweise als portable Steuervorrichtung ausgestaltet sein und kann beispielsweise ein mobiles Kommunikationsgerät, wie beispielsweise ein Mobilfunkgerät, umfassen. Auch tragbare Computer können umfasst sein, beispielsweise PDAs (Personal Digital Assistant). Besonders bevorzugt ist es jedoch, wenn die Steuervorrichtung selbst mindestens eine von dem medizinischen Benutzerelement unabhängige Messfunktion aufweist. Unter einer unabhängigen Messfunktion ist dabei eine Messfunktion, also die Erfassung einer physikalischen und/oder chemischen und/oder physiologischen Eigenschaft des Benutzers, zu verstehen, welche unabhängig von einer gegebenenfalls durchgeführten Messung des medizinischen Benutzerelements erfolgt. Dabei kann es sich zwar grundsätzlich um eine Messgröße handeln, welche ebenfalls von dem medizinischen Benutzerelement erfasst wird, welche jedoch von der medizinischen Steuervorrichtung unabhängig erfasst wird. Insbesondere kann es sich bei dieser Messfunktion um eine Messfunktion zur Erfassung mindestens einer Eigenschaft des Körpers des Benutzers handeln, beispielsweise einer Körperfunktion und/oder einer Analytkonzentration. Beispielsweise kann die Steuervorrichtung eine Steuervorrichtung sein, welche eingerichtet ist, um mittels mindestens eines Testelements, beispielsweise eines Teststreifens und/oder eines Testbandes und/oder einer Testscheibe, einen Analyten in einer Körperflüssigkeit qualitativ und/oder quantitativ nachzuweisen. Das medizinische System kann insbesondere eingerichtet sein, um mittels der Messfunktion der Steuervorrichtung eine Kalibrationsmessung durchzuführen, beispielsweise eine Kalibrationsmessung an Blutglukose. Insbesondere kann die Steuervorrichtung eingerichtet sein, um einen Analyten qualitativ oder quantitativ in einer Blutprobe, insbesondere einer Vollblut-Probe, nachzuweisen. So kann beispielsweise die Steuervorrichtung gleichzeitig ein Messgerät für Glukose aus Vollblut sein. Diese Messwerte können beispielsweise als Referenz-Messverfahren herangezogen werden. Auf diese Weise können beispielsweise Messsignale, welche beispielsweise in den persönlichen Daten enthalten sein können, ausgewertet werden, wobei bei der Auswertung eine Kalibrationsinformation verwendet wird, die von der Steuervorrichtung gewonnen wird.

Das medizinische Benutzerelement seinerseits kann auf verschiedene Weisen ausgestaltet sein und verschiedene medizinische Funktionen umfassen. So kann das medizinische Benutzerelement beispielsweise, wie oben bereits beschrieben, einen Sensor zum Nachweis mindestens eines Analyten in einer Körperflüssigkeit umfassen, insbesondere einen in ein Körpergewebe implantierbaren Sensor. Beispielsweise kann dieser Sensor in einem Continuous Monitoring Glucose System eingesetzt werden. Der Sensor kann beispielsweise, wie oben dargestellt, ein oder mehrere Disposables, ein oder mehrere Reusables, Auslese- und Datenmanagement-Komponenten umfassen.

Alternativ oder zusätzlich kann das medizinische Benutzerelement weiterhin eine Messvorrichtung zur Erfassung mindestens einer Körperfunktion umfassen. Diese Messvorrichtung kann beispielsweise einen oder mehrere der genannten Sensoren zum qualitativen und/oder quantitativen Nachweis des Analyten in der Körperflüssigkeit umfassen. Auch andere Körperfunktionen können jedoch grundsätzlich erfasst werden, wie beispielsweise Körpertemperatur, Blutdruck oder Ähnliches. Wiederum alternativ oder zusätzlich kann das medizinische Benutzerelement auch eine Medikationsvorrichtung umfassen, also eine Vorrichtung, welche in der Lage ist, um bestimmte Substanzen, beispielsweise Medikamente, an den Körper abzugeben. Als Beispiel können hier insbesondere Insulinpumpen genannt werden. Wiederum alternativ oder zusätzlich kann das medizinische Benutzerelement auch ein oder mehrere medizinische Aktorvorrichtungen mit mindestens einem Aktor zur Steuerung einer Körperfunktion umfassen. Als Beispiele sind hier Schrittmacher, Ventile oder Ähnliches zu nennen.

Bei implantierbaren Sensoren kann die Zuordnungskopplung vorzugsweise im nicht-implantierten Zustand erfolgen. Alternativ kann jedoch die Zuordnungskopplung beispielsweise auch derart eingerichtet sein, dass diese mit einem nicht-implantierten Teil des ansonsten implantierten Sensors erfolgt. Gerade im Fall implantierbarer Sensoren macht sich die oben beschriebene Ausgestaltung, bei welcher das medizinische Benutzerelement vorzugsweise keine eigene Aktorik aufweist, um dem Benutzer Zustände direkt zu signalisieren, insbesondere vorzugsweise keine Eingabe-Ausgabeschnittstelle, besonders vorteilhaft bemerkbar. Allgemein sei darauf hingewiesen, dass das medizinische Benutzerelement auch beispielsweise mehrere Funktionen umfassen kann, beispielsweise mehrere Messfunktionen oder kombinierte Messfunktionen und Aktorfunktionen.

Das medizinische System kann weiterhin eingerichtet sein, um einen automatischen Trennschritt zu ermöglichen. Durch diesen automatischen Trennschritt kann ein weiterer Austausch persönlicher Daten zwischen dem medizinischen Benutzerelement und der Steuervorrichtung verhindert werden. Dies kann insbesondere bedeuten, dass die Zuordnung zwischen dem medizinischen Benutzerelement und der Steuervorrichtung aufgehoben wird, also ein so genanntes Unpairing. Der Trennschritt kann beispielsweise dadurch initiiert werden, dass erneut die oben beschriebene Zuordnungskopplung herbeigeführt wird, beispielsweise gemäß einer oder mehrerer der oben beschriebenen Ausführungsformen. Insofern kann wiederum auf die obige Beschreibung verwiesen werden. Das System kann eingerichtet sein, dass bei der erneuten Herbeiführung der Zuordnungskopplung der Zuordnungsschritt erneut durchgeführt wird und in diesem Fall als Trennschritt interpretiert wird. Dementsprechend kann weitgehend auf die obige Beschreibung möglicher Optionen verwiesen werden. Beispielsweise kann eine erste Herbeiführung der Zuordnungskopplung als Initiierung eines Pairings interpretiert werden und eine zweite Herbeiführung der Zuordnungskopplung als Initiierung des Unpairings, wobei gegebenenfalls weitere Herbeiführungen von Zuordnungskopplungen alternierend als Initiierung von Pairing bzw. Unpairing interpretiert werden können. Das System kann weiterhin eingerichtet sein, um jeweils den aktuellen Zustand zwischen einem bestimmten medizinischen Benutzerelement und der Steuervorrichtung für den Benutzer erkennbar zu machen, beispielsweise über ein Anzeigenelement. So kann beispielsweise auf einem Display der Steuervorrichtung angezeigt werden, ob mit einem oder mehreren medizinischen Benutzerelementen, welches sich in einer Reichweite für einen Datenaustausch befinden, ein Pairing oder ein Unpairing besteht, das heißt ob ein Austausch persönlicher Daten stattfinden kann oder nicht. Alternativ kann ein automatischer Trennschritt jedoch grundsätzlich auch durch einen anderen Vorgang initiiert werden, beispielsweise durch Drücken einer Taste oder ähnliches.

Weitere mögliche Ausgestaltungen des medizinischen System betreffen den Austausch von Daten, beispielsweise der persönlichen Daten oder von Daten, welche die medizinischen Daten umfassen können. So soll die Bewegungsfreiheit des Benutzers durch den Austausch der Daten, welcher, wie oben beschrieben, drahtlos erfolgt, möglichst gering eingeschränkt werden. Dementsprechend sollte der Datenaustausch derart schnell erfolgen, dass alle zu übertragende Daten innerhalb der Zeit übertragen werden, innerhalb derer sich der Benutzer bei normalen Bewegungen an einer Steuervorrichtung vorbeibewegt, was auch als Datentransfer "by the way" beschrieben werden kann. Typische Bewegungsgeschwindigkeiten von Benutzern liegen ca. bei 1 m/s. Dementsprechend kann die Steuervorrichtung beispielsweise stationär und getrennt vom Benutzer aufgenommen sein, wohingegen das medizinische Benutzerelement mit dem Benutzer verbunden ist und sich mit diesem mitbewegt. Der Verbindungsaufbau und die Nutz-Datenübertragung sollten dementsprechend innerhalb dieser Zeit erfolgen, woraus sich typischerweise Datenübertragungsraten von mehr als 100kbit/s ergeben.

Da Funksysteme üblicherweise in physikalisch undefinierten Umfeldern arbeiten, sind Übertragungsstrecken in vielen Fällen gestört. Dies kann dazu führen, dass eine Übertragung bei Annäherung unter ungünstigen Umständen nicht vollständig erfolgt oder auch vollkommen gestört ist. Um dieses Risiko zu reduzieren, kann bei dem vorgeschlagenen medizinischen System gegebenenfalls ein zeitlicher Verlauf von persönlichen Daten, beispielsweise Messwerten, in Kompartimenten übertragen werden. Es wird beispielsweise vorgeschlagen, dass das medizinische System eingerichtet ist, um zumindest bei wiederholter kurzfristiger Annäherung zwischen der Steuervorrichtung und dem medizinischen Benutzerelement im medizinischen Betrieb einen wiederholten Austausch komplementärer Datensätze zu ermöglichen. Beispielsweise können Kurvenzüge, d.h. eine Mehrzahl von Datenpunkten, übertragen werden, wobei jeweils eine Teilmenge der Datensätze bei jedem Übertragungsvorgang übertragen wird. Diese Teilmenge kann grundsätzlich den gesamten Bereich des Kurvenzuges und/oder eines betrachteten Zeitintervalls abdecken, jedoch mit vergleichsweise geringer zeitlicher und/oder dynamischer Auflösung. Beispielsweise können einzelne Datensätze übertragen werden, welche in äquidistanten zeitlichen Abständen aufgenommen wurden. So ist es beispielsweise möglich, den Kurvenzug zwar nicht in voller Genauigkeit, aber qualitativ gut und vollständig darzustellen, auch wenn lediglich eine oder wenige Übertragungsvorgänge erfolgen. Bei der nächsten Annäherung kann dann gegebenenfalls eine weitere aber unterschiedliche Teilmenge der zu übertragenden Daten gesendet werden, gefolgt gegebenenfalls von weiteren Übertragungsvorgängen. So ermöglicht bereits ein erstes, relativ kurzes Datenpaket die Darstellung und/oder Auswertung einer vollständigen Kurve der bis dahin erfassten Datenpunkte, die mit jedem Datensatz genauer wird, bis zu einer vollständigen Übertragung des Datensatzes.

Wie oben dargestellt, wird neben dem medizinischen System weiterhin ein Verfahren zum Betrieb eines medizinischen Systems, insbesondere eines medizinischen Systems gemäß einer oder mehrerer der vorangehend beschriebenen Ausführungsformen, vorgeschlagen. Dabei wird eine Steuervorrichtung verwendet und mindestens ein medizinisches Benutzerelement. Das medizinische Benutzerelement und die Steuervorrichtung sind eingerichtet, um drahtlos Daten auszutauschen. Bei dem Verfahren wird ein automatischer Zuordnungsschritt durchgeführt, wobei durch den automatischen Zuordnungsschritt ein Austausch persönlicher Daten zwischen dem medizinischen Benutzerelement und der Steuervorrichtung freigegeben wird. Der automatische Zuordnungsschritt wird durch eine Zuordnungskopplung zwischen dem medizinischen Benutzerelement und der Steuervorrichtung automatisch initiiert. Die Zuordnungskopplung wird anschließend für einen medizinischen Betrieb des medizinischen Systems wieder getrennt. Für weitere mögliche Ausgestaltungen des Verfahrens kann auf die obige Beschreibung verwiesen werden.

Das vorgeschlagene medizinische System und das vorgeschlagene Verfahren weisen gegenüber bekannten Systemen und Verfahren zahlreiche Vorteile auf. So lassen sich mittels des vorgeschlagenen medizinischen Systems beispielsweise durch konsequente Plug-and-Play-Konzeptionen die genannten Nachteile bekannter Systeme, beispielsweise kontinuierlich messender Self-Monitoring-Diabetes-Systeme, weitgehend vermeiden. Das Plug-and-Play-Konzept kann sich dabei auf den gesamten interaktiven Handlungsablauf zwischen dem medizinischen System und dem Benutzer beziehen.

Der Benutzer kann insbesondere in die Lage versetzt werden, nur mit elementaren Handlungsschritten, wie beispielsweise einem Auspacken aus einer Primärverpackung, einem Vorbereiten der biologischen Applikation, einem Ansetzen des Systems am Körper und einem Durchführen einer Implantation, einen subkutanen kontinuierlichen Glukosemessvorgang zu starten und in Betrieb zu halten. Zum Ende eines Messzyklus kann dann eine Abnahme des Systems bzw. des medizinischen Benutzerelements manuell durchgeführt werden. Allgemein kann bei oder nach der Abnahme des medizinische Benutzerelements, beispielsweise dem oben beschriebenen Trennschritt, gegebenenfalls automatisch ein Datensicherungsschritt veranlasst werden, bei welchem beispielsweise Messdaten von dem Benutzerelement auf die Steuervorrichtung übertragen und dort gegebenenfalls gespeichert werden können.

Das medizinische System kann insbesondere weiterhin derart ausgestaltet sein, dass ein Einsetzen von Teilkomponenten, beispielsweise Datenträger, Batterien oder Ähnliches und/oder eine Dateneingabe und/oder ein Aktivieren und Initialisieren von Abläufen durch den Benutzer weitgehend entfallen kann. Das medizinische System und dessen Komponenten können beispielsweise derart ausgestaltet sein, dass diese lediglich in minimaler Weise vom Benutzer zu konfigurieren sind, beispielsweise lediglich durch Einstellungen von Landessprachen und Zeitzonen. Auch diese Schritte könnten jedoch optional beispielsweise durch die Feststellung von Ortskoordinaten, beispielsweise über eine GPS-Lokalisation und/oder über Funk-Zeitsysteme, automatisch vom medizinischen System oder einzelner Komponenten des medizinischen Systems vorgenommen werden. Der Bezug auf eine genaue Zeit kann gegebenenfalls therapeutisch essenziell sein, beispielsweise bei Diabetes hinsichtlich der Verabreichung von Wirkstoffen. Gerade bei Reisen kann daher ein automatisches Umstellen der Zeit, beispielsweise einer lokalen Zeit in Bezug auf eine absolute Zeit, wichtig sein. Das medizinische System kann eingerichtet sein, um eine derartige automatische Umstellung vorzunehmen.

Mittels des medizinischen Systems kann beispielsweise bei Self-Monitoring-Systemen, beispielsweise Diabetes-Systemen, eine eindeutige und fehlerfreie Zuordnung von Messwerten und Ergebnissen zu einem definierten Benutzer erfolgen. Dies wird zunehmend wichtiger, da derartige Systeme heute verstärkt räumlich verteilt sind und dementsprechend verstärkt in die Öffentlichkeit gelangen, beispielsweise im Rahmen von Arztpraxen, Kommunikationsmedien oder Ähnlichem.

Zudem bietet das medizinische System den Vorteil, dass nicht nur eine einfache Inbetriebnahme und Außerbetriebnahme von Systemkomponenten und ganzen medizinischen Systemen erfolgen kann, sondern dass dementsprechend auch auf einfache Weise eindeutige Zuordnungen zweier oder mehrerer Systemkomponenten zueinander durch einfache Handlungsabläufe erfolgen können. Außer der Eindeutigkeit der Zuordnung ist als Vorteil auch zu vermerken, dass der Benutzer in seiner Handlungsfreiheit und Bewegungsfreiheit durch das vorgeschlagene medizinische System nicht oder nur unwesentlich eingeschränkt wird.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche bzw. hinsichtlich ihrer Funktionen einander entsprechende Elemente.

Im Einzelnen zeigt:
- Figur 1: ein Beispiel eines medizinischen Benutzerelements in Form eines implantierba- ren Sensors;
- Figur 2: den implantierbaren Sensor während der Implantation in einer Schnittdarstel- lung von der Seite;
- Figur 3: ein erstes Ausführungsbeispiel eines "Pairing By Near Closing";
- Figur 4: ein zweites Ausführungsbeispiel eines "Pairing By Near Closing";
- Figur 5: ein Ausführungsbeispiel eines Pairings durch Verwendung einer Dockingstati- on;
- Figur 6: ein Pairing durch Aufnahme biometrischer Merkmale;
- Figur 7: eine Datenübertragung von einem bewegten Benutzer zu einer stationären oder mit einer anderen Geschwindigkeit bewegten Steuervorrichtung;
- Figur 8: ein Beispiel einer komplementären Datenübertragung; und
- Figur 9: eine schematische Darstellung einer Einkopplung eines Computers in ein me- dizinisches System.

### Ausführungsbeispiele

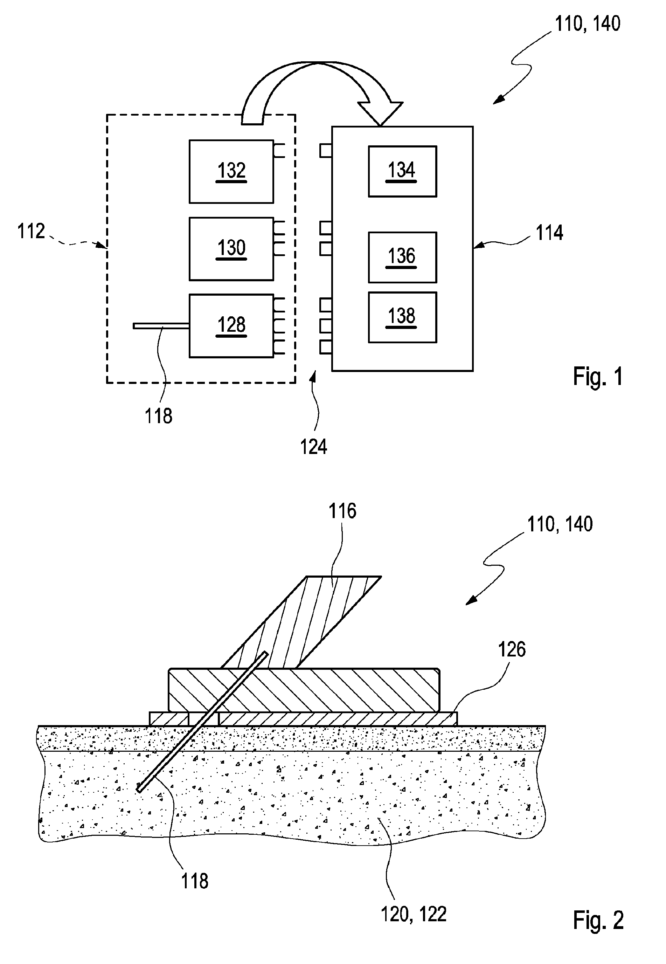

Ein erfindungsgemäßes medizinisches System wird im Folgenden exemplarisch am Beispiel eines kontinuierlichen Glukoseüberwachungssystems (Continuous Monitoring Glucose System) beschrieben. Ein derartiges System umfasst einen in den Figuren 1 und 2 symbolisch dargestellten Sensor 110, welcher eine Einweg-Einheit (Disposable) 112 und eine Mehrweg-Einheit (Reusable) 114 aufweist. In Figur 1 sind diese Einheiten 112, 114 schematisch dargestellt. Figur 2 ist ein Implantationsvorgang des Sensors 110 mittels einer Implantationshilfe 116, welche gegebenenfalls später wieder entfernt werden kann, dargestellt.

Der Sensor 110 umfasst ein in das Körpergewebe, beispielsweise ein interstitielles Gewebe, einschiebbares Sensor 118. Die Einweg-Einheit 112 und die Mehrweg-Einheit 114 sind dabei in Figur 2 nicht dargestellt. Beispielsweise kann die Mehrweg-Einheit 114 nach dem Implantieren des Sensorelements 118 in ein Körpergewebe 120 eines Benutzers 122 über eine Schnittstelle 124 (siehe Figur 1), insbesondere eine steckbare Hardware-Schnittstelle, mit der Einweg-Einheit 112 verbunden werden. Der Sensor 110 kann weiterhin ein Sensorpflaster 126 umfassen, mittels dessen der Sensor 110 auf einer Hautoberfläche des Benutzers 122 aufgeklebt werden kann.

Wie aus der schematischen Darstellung in Figur 1 hervorgeht, kann der Sensor 110, beispielsweise die Einweg-Einheit 112, beispielsweise neben einer Sensoreinheit 128 zur Aufnahme von Messdaten einen Energiespeicher 130 und gegebenenfalls einen Datenspeicher 132 zur Zwischenspeicherung von Sensordaten umfassen. Die Mehrweg-Einheit 114 verfügt vorzugsweise über keinen eigenen Energiespeicher und wird von der Einweg-Einheit 112 mit elektrischer Energie versorgt. Die Mehrweg-Einheit 114 kann beispielsweise ebenfalls einen Datenspeicher 134 umfassen sowie eine Schnittstelle 136 zum drahtlosen Datenaustausch mit einer Steuervorrichtung, beispielsweise eine Funkschnittstelle. Darüber hinaus kann eine vorzugsweise von der Schnittstelle 136 vollständige oder teilweise verschiedene Schnittstelle 138 vorgesehen sein, für welche für den im Folgenden beschriebenen automatischen Zuordnungsschritt verwendet werden kann, der im Folgenden auch als Pairing bezeichnet wird. Über diese Schnittstelle 138 kann beispielsweise die oben beschriebene Zuordnungskopplung erfolgen, über welche die Zuordnungsidentifikation ausgetauscht werden kann.

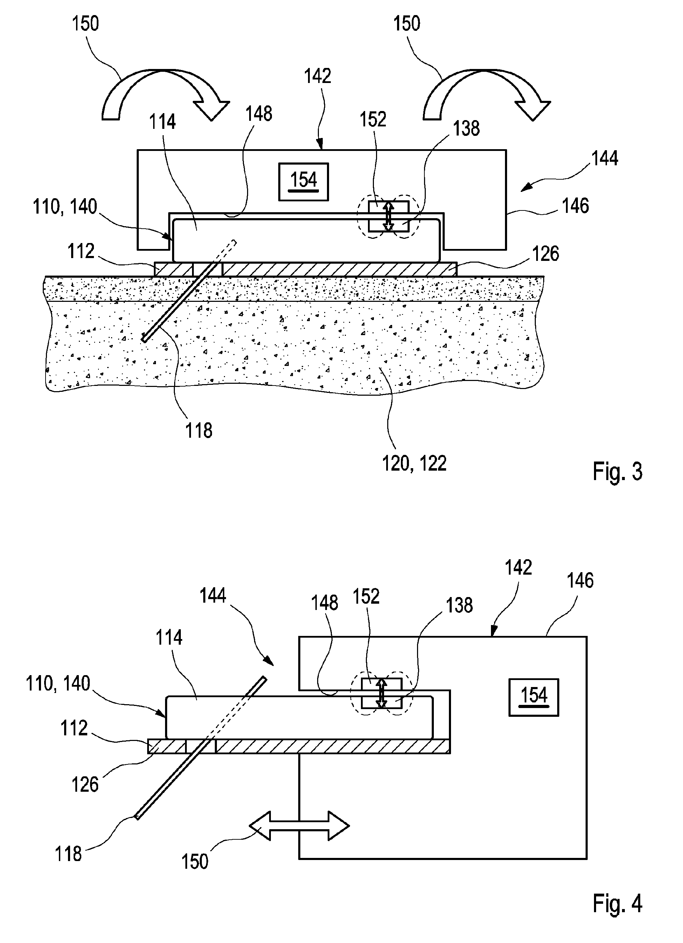

Der in den Figuren 1 und 2 dargestellte Sensor 110 bildet ein medizinisches Benutzerelement 140, welches einem Benutzer 122 zugeordnet ist und vorzugsweise von diesem Benutzer ständig mitgeführt wird, insbesondere unmittelbar am bzw. im Körper. Als elementarer Handhabungsschritt des Sensors 110 erfolgt im vorliegenden Beispiel das Auspacken des Disposables 112, das Verbinden dieses Disposables 112 mit dem Reusable 114 und das Insertieren des Sensorelements 118 in das Körpergewebe 120. Die so entstandene Funktionseinheit ist damit betriebsbereit und kann beispielsweise automatisch mit der Erfassung von Messwerten beginnen. Die beschriebene Einheit des Sensors 110 arbeitet dann vorzugsweise autark und kann beispielsweise persönliche Daten, insbesondere Messdaten, Zeiten, Ergebnisse oder Ähnliches, nach außen jedoch nicht sichtbar zu machen. Um diese Messwerte weiter zu verarbeiten und darzustellen, wird daher eine Steuervorrichtung 142 benötigt (siehe beispielsweise Figur 3). Mindestens eine derartige Steuervorrichtung 142 und das mindestens ein medizinische Benutzerelement 140 bilden gemeinsam ein medizinisches System 144. Die Steuervorrichtung 142 kann beispielsweise ein Messgerät umfassen, beispielsweise ein Blutglukosemessgerät. Alternativ oder zusätzlich kann diese auch einen Datenmanager und/oder weiterverarbeitende Computersysteme umfassen.

Der Datenaustausch vom Sensor 110, der in diesem Fall als Messwerterfassungsmodul dient, hin zur Steuervorrichtung 142, in diesem Fall dem Blutglukosemessgerät, muss derartig ausgestaltet sein, dass dieser eindeutig zuzuordnen ist. Es sollte auf jeden Fall vermieden werden, dass beispielsweise Daten eines Nachbarsystems in einen nichtauthentifizierten Speicher und/oder auf eine falsche Anzeigeeinheit gelangen. Damit Komponenten zum Austausch von privaten Daten berechtigt sind, sollte vor Betriebsbeginn des medizinischen Systems 144 ein bewusster Authentifizierungsschritt im Rahmen eines automatischen Zuordnungsschritts durchgeführt werden, welcher auch als Pairing bezeichnet wird. Bei diesem Schritt können beispielsweise eine oder mehrere Gerätenummern, insbesondere eindeutige Gerätenummern, beispielsweise der Steuervorrichtung 142 und/oder des Sensors 110, ausgetauscht werden. Diese Gerätenummern und/oder andere Arten von Zuordnungsidentifikationen können beispielsweise in einem oder mehreren Datenspeichern in der Steuervorrichtung 142 und/oder dem Sensor 110 hinterlegt werden, und es können gegebenenfalls Verknüpfungen hergestellt werden. Beispielsweise kann eine eindeutige Gerätenummer des Sensors 110 in Datenspeicher 132 hinterlegt sein, welche gegebenenfalls ausgetauscht werden kann und mit der beispielsweise eine Verknüpfung in der Steuervorrichtung 142 hergestellt werden kann, so dass nach Durchführung des automatischen Zuordnungsschritts beispielsweise die Steuervorrichtung 142 weiß, dass persönliche Daten mit diesem speziellen Sensor 110 ausgetauscht werden können. Der automatische Zuordnungsschritt soll zwar vom Benutzer 122 sehr bewusst durchgeführt werden, soll jedoch nicht das Fehlerpotenzial einer manuellen Nummern- oder Codeeingabe aufweisen.

Bei herkömmlichen Punkt-zu-Punkt-Steckverbindungen ist dies leicht zu gewährleisen, da der Benutzer 122 in diesem Fall die Steckverbindung aktiv und wissentlich herstellt und dann bei richtiger Konstruktion von außen keine Signale eingespeist oder abgegriffen werden können. Bei optischen Verbindungen und vor allem bei Funkverbindungen ist dies jedoch nicht gegeben. Kabellose Verbindungen und im Speziellen Funkverbindungen weisen jedoch trotzdem zahlreiche Vorteile hinsichtlich der Flexibilität und Modularität von Produktkonzepten auf. Bei dem vorgeschlagenen Sensor 110 ist daher die oben genannte Schnittstelle 136 für einen drahtlosen Datenaustausch, insbesondere als Funkschnittstelle, eingerichtet.

In den Figuren 3 bis 6 sind verschiedene Beispiele von Ausführungsformen erfindungsgemäßer medizinischer Systeme 144 und Ausführungsformen automatischer Zuordnungsschritte dargestellt. In jedem dieser Fälle wird eine Zuordnungskopplung zwischen der Steuervorrichtung 142 und dem mit dieser zu koppelnden medizinischen Benutzerelement 140 erzeugt, über welche mindestens eine Zuordnungsidentifikation zwischen der Steuervorrichtung 142 und dem medizinischen Benutzerelement 140 ausgetauscht werden kann.

So zeigen die Figuren 3 und 4 verschiedene Ausführungsbeispiele eines medizinischen Systems 144, bei welchen das medizinische Benutzerelement 140 und die Steuervorrichtung 142 von ihrer äußeren Gestalt her derart eingerichtet sind, dass diese relativ zueinander in eine definierte vorübergehende Kopplungsausrichtung gebracht werden können, in welcher die genannte Zuordnungskopplung vorliegt und damit der Zuordnungsschritt initiiert wird, bei welchem wiederum die mindestens eine Zuordnungsidentifikation ausgetauscht werden kann. In beiden Fällen kann die Steuervorrichtung 142 beispielsweise wieder als Blutglukosemessgerät ausgestaltet sein. Die Steuervorrichtung 142 weist ein Gehäuse 146 auf, welches über eine der äußeren Gestalt des Sensors 110 angepasste Vertiefung 148 verfügt. Mittels dieser Vertiefung 148 ist ein Formschluss zwischen dem Sensor 110 und der Steuervorrichtung 142 möglich, welcher von dem Benutzer 122 bewusst und vorübergehend herbeigeführt werden kann, um die Zuordnungskopplung zu erzeugen und damit den oben beschriebenen Zuordnungsschritt auszulösen. Die Ausführungsbeispiele in den Figuren 3 und 4 unterscheiden sich dabei dahingehend, dass in Figur 3 der Sensor 110 bereits in dem Körpergewebe 120 des Benutzers 122 implantiert ist und dass die Steuervorrichtung 142 lediglich von oben her auf den außen zugänglichen Teil des Sensors 110 aufgesetzt werden kann, beispielsweise lediglich auf die Mehrweg-Einheit 114. Diese kann beispielsweise einen quadratischen oder rechteckigen Grundriss aufweisen, dem der Grundriss der Vertiefung 148 entsprechen kann, so dass der Benutzer eine Zuordnungsorientierung ohne größere Schwierigkeiten herbeiführen kann. In dem Ausführungsbeispiel gemäß Figur 4 hingegen ist die Vertiefung 148 als Tasche ausgestaltet, in welche der Sensor 110 mit seiner Schmalseite eingesteckt werden kann.

In beiden Fällen wird der Zuordnungsschritt durch die in den Figuren 3 und 4 dargestellte Zuordnungskopplung mit der dargestellten Zuordnungsorientierung initiiert. Symbolisch ist in beiden Fällen der Zuordnungsschritt bzw. dessen Initiierung durch Herstellung der Zuordnungskopplung mit der Bezugsziffer 150 bezeichnet. Vor Durchführung des Zuordnungsschritts 150 befinden sich das medizinische Benutzerelement 140 und die Steuervorrichtung 142 in einem nicht zugeordneten Zustand ("unpaired"), wohingegen sich nach dem Zuordnungsschritt 150 die beiden Elemente 140, 142 in einem zugeordneten Zustand ("paired") befinden.

Wie oben dargestellt, verfügt das medizinische Benutzerelement 140, beispielsweise der Sensor 110, insbesondere über eine separate Schnittstelle 138 für den Zuordnungsschritt. Diese separate Schnittstelle 138 ist vorzugsweise als nicht-galvanische Schnittstelle für einen nicht-galvanischen Datenaustausch eingerichtet. In analoger Weise kann die Steuervorrichtung 142 über eine Schnittstelle 152 verfügen, welche speziell für den Zuordnungsschritt konzipiert ist und welche vorzugsweise getrennt ausgebildet ist von einer optionalen weiteren Schnittstelle 154 für den drahtlosen Datenaustausch, beispielsweise eine Funkschnittstelle. In der dargestellten Kopplungsorientierung können die Schnittstellen 138, 152 jeweils derart zueinander orientiert sein, dass diese die Zuordnungsidentifikation austauschen können. Auf diese Weise kann der Zuordnungsschritt initiiert und durchgeführt werden.

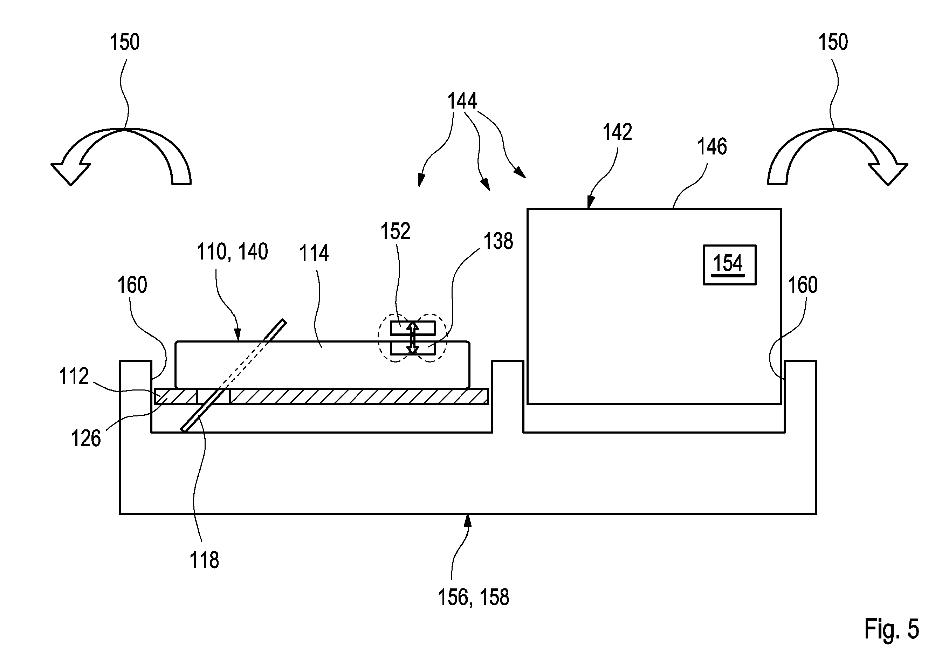

In Figur 5 ist ein weiteres Ausführungsbeispiel des medizinischen Systems 144 dargestellt, bei welchem die Zuordnungskopplung mittels eines Koppelelements 156 in Form einer Dockingstation 158 erfolgt. In diesem Fall weist die Dockingstation 158 eine oder zwei Schnittstellen 160 auf, mit welcher das medizinische Benutzerelement 140 und die Steuervorrichtung 142 nacheinander oder gleichzeitig mechanisch und/oder elektrisch verbunden werden können. Durch diese Schnittstellen 160 kann beispielsweise eine rein mechanische Orientierung der Steuervorrichtung 142 und des medizinischen Benutzerelements 140 zueinander erfolgen, beispielsweise für einen optimierten Austausch der Zuordnungsidentifikation. In Figur 5 ist dabei die Schnittstelle 152 der Steuervorrichtung 142 lediglich symbolisch angedeutet. Der Austausch der Zuordnungsidentifikation kann direkt von der Schnittstelle 138 zu der Schnittstelle 152 erfolgen, wie in Figur 5 angedeutet, oder beispielsweise über eine dritte Schnittstelle, welche Bestandteil der Dockingstation 158 sein kann und welche auch mehrteilig ausgebildet sein kann. Auf diese Weise kann durch die Dockingstation 158 als Koppelelement 156 die Zuordnungsinformation ausgetauscht werden.

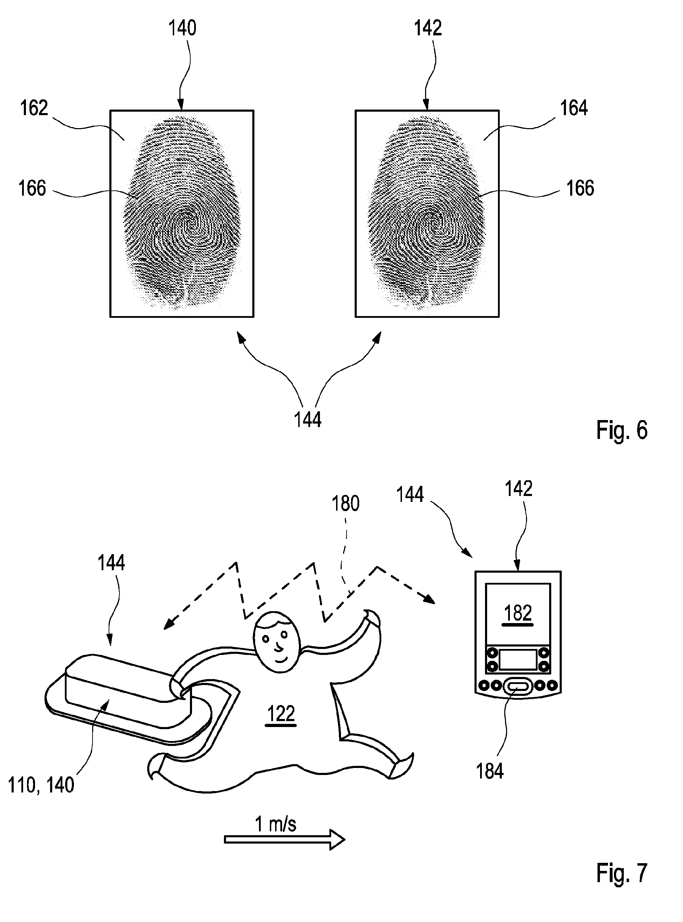

Eine dritte Möglichkeit der Zuordnungskopplung zwischen dem medizinischen Benutzerelement 140 und der Steuervorrichtung 142 ist in Figur 6 symbolisch dargestellt. Dabei weist sowohl das medizinische Benutzerelement 140 als auch die Steuervorrichtung 142 jeweils eine Erfassungsvorrichtung 162 bzw. 164 zur Erfassung eines biometrischen Merkmals des Benutzers auf. Das biometrische Merkmal ist dabei in Figur 6 symbolisch mit der Bezugsziffer 166 bezeichnet und kann beispielsweise einen Fingerabdruck umfassen. Die Erfassungsvorrichtung 162, 164 können dementsprechend beispielsweise einen Fingerabdruckscanner, beispielsweise in Form eines Scanner-Arrays und einer Scanner-Zeile, umfassen. Auch andere biometrische Merkmale 166 können eingesetzt werden. Der Zuordnungsschritt wird initiiert, indem der Benutzer nacheinander seinen Fingerabdruck bzw. das andere biometrische Merkmal 166 auf die Erfassungsvorrichtung 162 bzw. 164 aufbringt. Stimmen die biometrischen Merkmale 166 überein, so fungieren diese als Zuordnungsinformation und bewirken ein automatisches Pairing zwischen der Steuervorrichtung 142 und dem medizinischen Benutzerelement 140. Weist das medizinische System 144 derartige Erfassungsvorrichtungen 162, 164 auf, so kann gegebenenfalls auf separate Schnittstellen 138, 152 für den Zuordnungsschritt verzichtet werden, da die Erfassungsvorrichtungen 162, 164 in diesem Fall selbst als derartige Schnittstellen wirken.

Die in den Figuren 3-6 dargestellten möglichen Ausgestaltungen stellen nur eine Auswahl von Möglichkeiten dar, wie durch die Zuordnungskopplung ein Zuordnungsschritt initiiert werden kann. So wird in den Figuren 3 und 4 beispielsweise die Kombination einer eindeutigen und bewussten manuellen Handlung des Benutzers 122 durch ein sogenanntes "Near Closing" erreicht. Hierzu kann der Benutzer 122 beispielsweise den Sensor 110, beispielsweise einen Patch-förmigen Sensor 110, so nahe an die Steuervorrichtung 142, beispielsweise das Blutglukosemessgerät, heranbringen, dass ein unbeabsichtigter körperlicher Kontakt mit einem unautorisierten Modul quasi unmöglich ist. Dies kann, wie in den Figuren 3 und 4 angedeutet, beispielsweise auch durch ein kurzzeitiges zumindest teilweises Einstecken des medizinischen Benutzerelements 140 in die Vertiefung 148, beispielsweise eine Gehäusetasche am Blutglukosemessgerät, erfolgen. Auch eine andere geometrische Ausgestaltung des Benutzerelements 140 und/oder der Steuervorrichtung 142 zur Verhinderung einer unbeabsichtigten Zuordnungskopplung ist grundsätzlich möglich. So kann beispielsweise eine unbeabsichtigte Zuordnungskopplung allgemein durch mechanische Formen der Steuervorrichtung 142 und/oder des Benutzerelements 140, beispielsweise Schloss-Schlüssel-Formen, unterbunden werden.

Nach Durchführung des Zuordnungsschritts kann dann diese Zuordnungskopplung wieder aufgehoben werden, beide Komponenten 140, 142 können wieder getrennt werden, und es kann ein normaler medizinischer Betrieb initiiert werden. In diesem medizinischen Betrieb können beispielsweise persönliche Daten, beispielsweise Messdaten des Sensors 110, zwischen dem medizinischen Benutzerelement 140 und der Steuervorrichtung 142 ausgetauscht werden, beispielsweise über die Schnittstellen 136 und 154. Alternativ kann, wie anhand von Figur 5 gezeigt, auch eine Zuordnung über die Dockingstation 150 erfolgen, in welche beispielsweise das medizinische Benutzerelement 140 und die Steuervorrichtung 142 kurzfristig eingebracht, beispielsweise gestellt, werden können. Kommt ein solcher Formschluss zustande, so können beispielsweise Daten in digitaler Form zwischen den einander zuzuordnenden (d.h. zu pairenden) Modulen 140, 142 ausgetauscht werden, also beispielsweise eine Zuordnungsidentifikation, welche den Pairingvorgang durchführt, bestätigt und abschließt. Die beiden Komponenten 140, 142 können nun wieder physikalisch getrennt werden und sind von nun an einander zugeordnet (gepairt). Diese Zuordnung kann beispielsweise solange erfolgen, bis ein aktiver "Unpairing-Schritt" erfolgt, also Trennvorgang, beispielsweise durch erneutes Near Closing, beispielsweise analog zu einer oder mehreren der Ausführungsformen der Figuren 3-6.

Da es sich beispielsweise bei dem Sensor 110 um ein hochohmiges elektrochemisches Sensorsystem handeln kann, ist ein Datenaustausch über galvanische elektrische Verbindungen funktional in der Regel kritisch. Eine elektrisch hermetische Kapselung des Sensors 110 ist daher in der Regel anzustreben. Vorzugsweise sind daher eine oder beide der Schnittstellen 136, 138 für einen Datenaustausch mittels elektromagnetischer Felder eingerichtet, insbesondere die Schnittstelle 138. Auch die Schnittstelle 136, welche auch ganz oder teilweise mit der Schnittstelle 138 bauteilidentisch ausgestaltet sein kann, ist jedoch vorzugsweise derart ausgestaltet. Der Datenaustausch auf nicht-galvanische Weise kann beispielsweise über räumlich eng gekoppelte Drahtspulen (d.h. insbesondere induktiv) und/oder über isolierte Kondensatorplatten (d.h. beispielsweise kapazitiv) oder mittels RFID erfolgen. Alternativ oder zusätzlich kann auch ein optischer Datenaustausch (beispielsweise über eine Lichtschranke) erfolgen, was einen physikalischen Sichtkontakt voraussetzt. Sämtliche dieser Verfahren, welche für den Zuordnungsschritt verwendet werden können und welche eine entsprechende Einrichtung der Schnittstelle 138 für den Zuordnungsschritt erforderlich machen, verhindern weitgehend ein unbeabsichtigtes Pairing mit zufällig in der Nähe befindlichen Komponenten.

Vorzugsweise ist das medizinische System 144 derart eingerichtet, dass, außer der vorangehend beschriebenen Handlungen, keine weiteren Handlungen durch den Benutzer 122 durchzuführen sind. Derartige weitere Handlungen können jedoch optional vorgesehen sein. So kann beispielsweise zur weiteren Sicherung des Verfahrens der Zuordnungsschritt auch durch den Gebrauch einer weiteren, unabhängigen Funktion, wie beispielsweise das Drücken einer Taste (beispielsweise an der Steuervorrichtung 142 und/oder am medizinischen Benutzerelement 140), initiiert oder freigegeben werden. Eine weitere Möglichkeit, um die Wahrscheinlichkeit einer Fehlcodierung bzw. Fehlzuweisung zu senken, ist ein definierter zeitlicher Code bei einer Annäherung der beiden Komponenten 140, 142. Eine eindeutige Zuordnung ermöglicht auch die anhand der Figur 6 beschriebene Verknüpfung über biometrische Merkmale 166, beispielsweise des Benutzers 122, wie beispielsweise Fingerabdrücke, Irismuster oder Ähnliches. Diese Ausgestaltung bedeutet allerdings in der Regel einen vergleichsweise großen apparativen Aufwand, der aber im Rahmen der zunehmenden Verbreitung derartiger Sicherheitssysteme (beispielsweise der Verfügbarkeit kostengünstiger Erfassungsvorrichtungen 162, 164) sowohl vom Volumen her akzeptabel als auch wirtschaftlich in geeigneten Größenordnungen liegt.

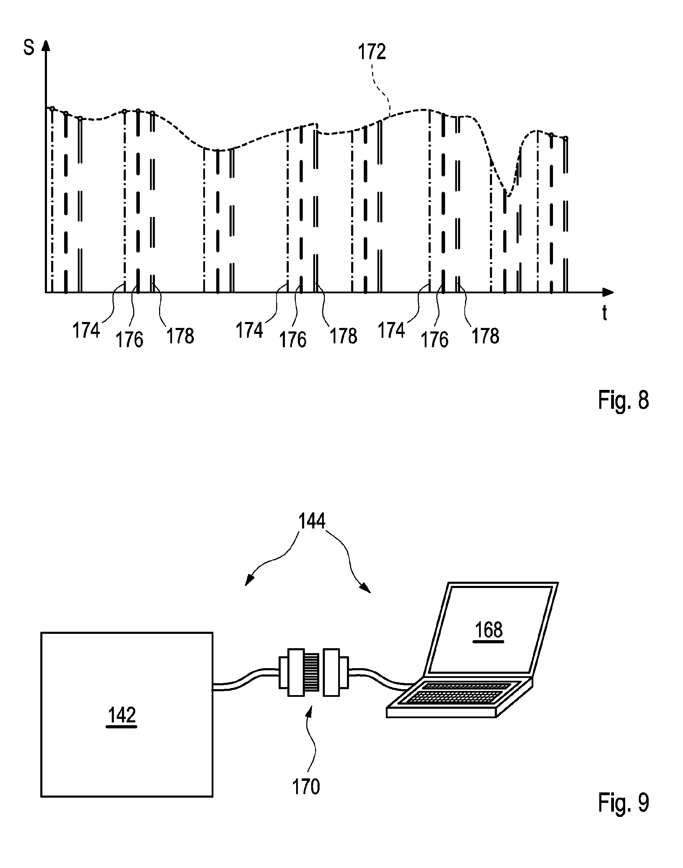

Die Anzahl einander zuzuordnender Komponenten des medizinischen Systems 144 ist grundsätzlich nicht begrenzt bzw. vorgeschrieben. So kann das medizinische System 144 auch ganz oder teilweise als medizinisches Netzwerk ausgestaltet sein und kann optional beispielsweise ein Datenloggermodul für Evaluierungszwecke aufweisen. Später kann, wie beispielsweise in Figur 9 angedeutet, ein Benutzer die von seinem Sensor 110 bzw. medizinischen Benutzerelement 140 gesammelten und vorzugsweise in der Steuervorrichtung 142 zumindest zwischengespeicherten persönlichen Daten direkt an ein Computersystem, beispielsweise ein PC-System 168, "uploaden", beispielsweise ein PC-System 168 eines Arztes. Dies kann beispielsweise, wie in Figur 9 angedeutet, über eine zusätzliche Schnittstelle 170 erfolgen, die hier exemplarisch als drahtgebundene Schnittstelle gezeigt ist, beispielsweise als RS232-Schnittstelle. Auf diese Weise kann das PC-System 168 beispielsweise in das medizinische System 144 eingebunden werden, beispielsweise kurzfristig. Diese Einbindung kann beispielsweise ebenfalls ein Pairing erforderlich machen, kann jedoch - insbesondere bei Verwendung drahtgebundener Schnittstellen 170 - auch ohne Zuordnungsschritt erfolgen.

Das medizinische System 144 kann auch optional zumindest teilweise als offenes System ausgestaltet sein und eine Interoperabilität ermöglichen. So kann beispielsweise ein Arzt sich grundsätzlich in der Regel nicht auf alle möglichen patientenspezifischen oder produktspezifischen Pairing-Schnittstellen einstellen, da beispielsweise eine Vielzahl von konkurrierenden Produkten existieren kann. Dementsprechend kann das medizinische System 144 beispielsweise die Einbindung einer besonders autorisierten Person oder einer besonders autorisierten Systemkomponente, beispielsweise eines Arztes und/oder eines Arztcomputers, ermöglichen, welche einen Sonderstatus genießen kann. So kann beispielsweise ein Spezialmodus "Arzt" vorgesehen seien, beispielsweise in einem Funksystem. In diesem Spezialmodus könnte sich ein Pairing mit der besonders autorisierten Person und/oder der besonders autorisierten Systemkomponente beispielsweise auf Software beschränken. Dies könnte beispielsweise derart erfolgen, dass eine herstellerübergreifende Konvention getroffen wird, welche jedes medizinische System 144 und/oder dessen Komponenten verstehen. Diesbezüglich kann beispielsweise auf den oben zitierten Standard IEEE 1073 verwiesen werden, welcher somit mit einem erfindungsgemäßen medizinischen System 144 kombiniert werden kann.

Nach erfolgtem Pairing, beispielsweise nach Durchführung der anhand der Figuren 3-6 beschriebenen Schritte, tauschen einander zugeordnete Komponenten des medizinischen Systems 144 vorzugsweise automatisch Daten aus, sobald physikalisch eine Übertragung möglich ist. Bei typischen Sensoren 110 kann dies beispielsweise dann erfolgen, wenn eine Annäherung zwischen den beiden Schnittstellen 136, 154 auf mehr als ca. 1m erfolgt. Dieser Abstand kann beispielsweise durch beliebige Kleidungsschichten hindurch bestehen. Physikalisch ist eine Übertragung grundsätzlich ohne räumliche Ausrichtung und damit durch verschiedenste Materialien hindurch praktisch nur durch die Verwendung von Funkwellen möglich. Grundsätzlich sind jedoch auch optische Datenübertragungen in diesem medizinischen Betrieb zumindest begrenzt realisierbar.

Nähern sich zwei möglicherweise miteinander kommunizierende Komponenten 140, 142 eines medizinischen Systems einander an, so kann das medizinische System 144 insbesondere derart eingerichtet sein, dass dieses automatisch überprüft, ob eine Zuordnung dieser Komponenten 140, 142 zueinander besteht, also ob diese für den Austausch persönlicher Daten autorisiert sind, insbesondere durch einen vorangegangenen Pairing-Schritt. Dies kann beispielsweise über Kommunikationsprotokolle erfolgen, beispielsweise gemäß dem OSI-Standard. So kann beispielsweise über diese Kommunikationsprotokolle ein stabiler Kontakt aufgebaut werden, und es kann geprüft werden, ob eine Autorisierung (Pairing) vorliegt, und die spezifischen Nutzdaten können dann, falls dies der Fall ist, gegebenenfalls unidirektional oder bidirektional ausgetauscht werden.

Um einer möglichst geringen Einschränkung der Bewegungsfreiheit des Benutzers 122 entgegen zu kommen, sollte im medizinischen Betrieb, also nach Durchführung des Pairing-Schritts bzw. des Zuordnungsschritts, ein Datenaustausch zwischen den einander zugeordneten Komponenten 140, 142 des medizinischen Systems 144 so schnell erfolgen, dass vorzugsweise alle zu übertragenden Daten innerhalb der Zeit übertragen werden, innerhalb derer sich ein Benutzer 122 normalerweise an einer nicht mit dem Benutzer 122 mitbewegten Steuervorrichtung vorbeibewegt. Diese Datenübertragung kann auch als "Datentransfer By The Way" bezeichnet werden und ist symbolisch in Figur 7 dargestellt. Beispielsweise kann sich, wie in Figur 7 angedeutet, der Benutzer 122 mit einer Geschwindigkeit von 1m/s relativ zu der Steuervorrichtung 142 bewegen. Dies bedeutet, dass der Verbindungsaufbau und die Übertragung persönlicher Daten, wie beispielsweise Nutzdaten, innerhalb dieser Zeit erfolgen sollte, woraus sich, wie oben dargestellt, beispielsweise Übertragungsraten von vorzugsweise mehr als 100 kbit/s ergeben sollten. Da Funksysteme in physikalisch undefinierten Umfeldern arbeiten, sind jedoch die Übertragungsstrecken oft gestört. Dies führt dazu, dass eine Übertragung bei Annäherung unter ungünstigen Umständen nicht vollständig erfolgt oder auch vollkommen gestört ist. Um dieses Risiko zu reduzieren, kann das medizinische System 144 beispielsweise derart eingerichtet sein, dass die persönlichen Daten durch einmaligen oder mehrmaligen Austausch komplementärer Datensätze übertragen werden. So können beispielsweise zeitliche Verläufe von Messwerten in Kompartimenten übertragen werden. Dies ist in Figur 8 symbolisch dargestellt. Aufgetragen ist dort eine Menge von Signalen S, beispielsweise Messwerten, als Funktion eines Messzeitpunktes t, beispielsweise der Zeitpunkte, zu welchen die Messdaten von dem Sensor 110 aufgenommen wurden. Auch andere Arten von Datensätzen als zeitliche Datensätze sind jedoch grundsätzlich denkbar.

Ein Kurvenzug 172 verbindet alle diskreten Datensätze in Figur 8 und bildet so den zeitlichen Verlauf des erfassten Signals als Funktion der Zeit t (d.h. S=f(t)) über einen gesamten Beobachtungszeitraum T ab. Der Beobachtungszeitraumes T ist in N Zeitintervalle, beispielsweise äquidistante Zeitintervalle, aufgeteilt. Die Datensätze in diesen Zeitintervallen, sind an den sich jeweils wiederholenden Positionen in den Intervallen in Figur 8 beispielhaft mit den Bezugsziffern 174, 176 und 178 bezeichnet.

Unter einem Datensatz kann dabei beispielsweise eine von einem so genannten Frame (Anfang- und/oder Enddefinition) eingefasste Menge alphanumerischer Zeichen verstanden werden. Beispielsweise können die in Figur 8 mit den Bezugsziffern 174, 176 und 178 bezeichneten Teil-Datensätze jeweils einen binären Spannungswert (S), die zugehörige Zeit (t) und optional weitere Elemente, beispielsweise ein oder mehrere Sicherungsmerkmalen wie ein CRC- Zeichen, umfassen. Unter einem Kurvenzug 172 kann hingegen eine Verbindung aller in den Datensätzen bzw. Teil-Datensätzen beschriebenen Positionen (S/t) in einem Koordinatensystem verstanden werden, was letztlich einer geschlossen Darstellung der Funktion S=f(t) gleichkommt.

Beispielsweise kann das medizinische System 144 derart eingerichtet sein, dass dieses zunächst bei einer Annäherung zwischen Benutzer 122 und Steuervorrichtung 142 den ersten Teil-Datensatz 174 der Intervalle 1 bis N drahtlos übermittelt. Der drahtlose Datenaustausch ist in Figur 7 symbolisch mit der Bezugsziffer 180 bezeichnet. Verbleibt anschließend noch Zeit während der Annäherung, so können weitere Teil-Datensätze 176, 178 der Intervalle 1 bis N übertragen werden. Alternativ können diese jedoch auch zu späteren Zeitpunkten, d.h. bei späteren Annäherungen, an die Steuervorrichtung 142 übermittelt werden.

Auf diese Weise ist es möglich, den Kurvenzug zwar zunächst nicht in voller Genauigkeit, aber qualitativ gut und vollständig durch den ersten Teil-Datensatz 174 darzustellen, was gegebenenfalls therapeutisch wichtiger sein kann (z.B. bei einer Unterzuckerung) als eine hohe zeitliche und/oder dynamische Auflösung. Verbleibt dann noch Zeit oder im Rahmen einer nächsten Annäherung, so können dann weitere, unterschiedliche Teil-Datensätze 176, 178 übermittelt werden. So ermöglicht schon ein erstes, relativ kurzes Datenpaket in Form eines Teil-Datensatzes 174 eine Darstellung einer gut angenäherten Messkurve des Kurvenzuges 172, beispielsweise auf einem Anzeigenelement 182 der Steuervorrichtung 142. Mit jeder Übermittlung eines Teil-Datensatzes 174, 176, 178 wird dann die Darstellung genauer, bis zur vollständigen Übertragung des Kurvenzuges 172. Zwischen einer ersten Annäherung zu einem Zeitpunkt t₁ und einer weiteren Annäherung zu einem Zeitpunkt tₙ kann grundsätzlich beliebig viel Zeit verstreichen, so dass die Datensatzerfassung fortgeschritten ist und die Anzahl der Intervalle sich auf N + M erhöht hat. Die Intervalle N+1 bis M, können beispielsweise bei einer zweiten Annäherung zuerst mit den Teil-Datensätzen 174 oder gleich mit den Teil-Datensätzen 176, genauso wie die Intervalle kleiner N, aufgefüllt werden. Alternativ sind aber auch andere Strategien der Datenorganisation einer fortlaufenden Datenerfassung möglich.

Die Steuervorrichtung 142 ist in Figur 7 symbolisch als PDA dargestellt, mit dem Anzeigenelement 182 in Form eines Displays und einem oder mehreren Bedienelementen 184. Alternativ oder zusätzlich kann die Steuervorrichtung 142 jedoch auch beispielsweise als Blutglukosemessgerät ausgestaltet sein, wie oben bereits beschrieben wurde. So kann die Steuervorrichtung 142 beispielsweise als Messgerät für Glukose aus Vollblut ausgestaltet sein. Die Messwerte dieses Messgerätes können beispielsweise als Referenz-Messverfahren herangezogen werden, beispielsweise um das medizinische System 144 zu eichen und/oder zu kalibrieren. Das Vollblut-Messgerät kann beispielsweise, ähnlich zum Sensor 110, einen Datenträger benötigen, welcher chargenspezifische oder Lot-spezifische Daten von Testelementen an das Messgerät mitteilt. Dies kann beispielsweise durch manuelles Einstecken eines elektronischen Datenträgers in das Messgerät erfolgen.

Die Steuervorrichtung 142 kann jedoch, alternativ oder zusätzlich, auch als Datenmanager ausgestaltet sein und beispielsweise mit einem separaten Blutglukosemessgerät gekoppelt werden. Dieses separate Blutglukosemessgerät kann dann mittels einer konventionellen Technik oder auch mit einer entsprechenden Plug-and-Play-Technik, beispielsweise gemäß dem erfindungsgemäßen Verfahren, in das medizinische System 144 eingefügt werden.

Mehrere komplette Datensätze zu medizinischen Benutzerelementen 140, beispielsweise zu Disposable/Reusable-Systemen, können in der Steuervorrichtung 142 gespeichert werden. Dort können diese beispielsweise mittels Echtzeit-Daten, den Blutglukose-Daten und weiteren so genannten Events zeitlich korreliert werden. Die Datensätze können, ebenfalls beispielsweise über einen in einem Plug-and-Play-Schritt autorisierten Computer, beispielsweise das PC-System 168 in Figur 9, ausgelesen und gegebenenfalls ganz oder teilweise weiterverarbeitet werden.

Wie oben dargestellt, weist das medizinische Benutzerelement 140 vorzugsweise keine eigene Aktorik und/oder Eingabe-/Ausgabe-Schnittstelle auf, beispielsweise kein eigenes Display, um dem Benutzer 122 Zustände direkt zu signalisieren, die dann gegebenenfalls von diesem interpretiert werden bzw. sogar von diesem interpretiert werden müssten. So ist es bevorzugt, wenn verstärkt und aktiv vom medizinischen System 144 dafür gesorgt wird, dass Fehler nicht zu falschen Messwerten führen. Dies kann beispielsweise weitgehend mittels sogenannter "Failsafe"-Funktionen realisiert werden, beispielsweise mit einer Sensor-Elektrodenüberwachung, einer Überwachung hinsichtlich einer Kontaktunterbrechung, einer Überwachung der Betriebsspannungen, einer Überwachung von Signalmustern oder ähnlichen Fehlerüberwachungen. Ist das medizinische System 144 in seinen Grundfunktionen noch arbeitsfähig, beispielsweise hinsichtlich der Signalerfassung durch den Sensor 110 (beispielsweise einem optischen und/oder elektrochemischen Analytnachweis), einer Spannungsversorgung oder einer Telemetrie, so können beispielsweise die Zustände des medizinischen Benutzerelements 140, beispielsweise des Sensors 110, zusammen mit den Datensätzen der persönlichen Daten abgespeichert werden, beispielsweise in dem Datenspeicher 132 und/oder dem Datenspeicher 134. Dieser Status kann dann, gegebenenfalls gemeinsam mit den persönlichen Daten, beispielsweise dem Datensatz und/oder Kurvenzug 172, abgespeichert, zur Steuervorrichtung 142 übertragen und dort gegebenenfalls auch auf dem Anzeigenelement dargestellt werden. Da jedoch insbesondere der Zeitpunkt der drahtlosen Datenübertragung nicht zeitnah zum eigentlichen Ereignis sein muss, welches zur Aufnahme des Datensatzes und/oder Kurvenzugs 172 geführt hat oder zum Zeitpunkt einer Fehlfunktion, so können beispielsweise Messdaten, die mit Fehlermeldungen im Status behaftet sind, von einer Anzeige ausgeschlossen werden. Gegebenenfalls können auch Hinweise an einen Benutzer ausgegeben werden, beispielsweise wiederum mittels des Anzeigenelements 182.

Im weitesten Sinne können unter dem oben beschriebenen Plug-and-Play-Verfahren auch andere Überwachungen vorgenommen werden. So könnte beispielsweise auch eine Überwachung eines ordnungsgemäßen Sitzes des Sensors 110, beispielsweise einer Sensor/Patch-Einheit, erfolgen, beispielsweise eine ordnungsgemäße Haftung des Sensorpflasters 126 auf einer Hautoberfläche des Benutzers 122. Dies ist insbesondere dann zu befürworten, wenn Langzeit-Sensoren 110 eingesetzt werden, welche beispielsweise über eine Woche hinweg oder mehr, Messergebnisse liefern sollen. Auf diese Weise kann beispielsweise eine Sensoreinstichstelle und/oder ein Hautbereich unterhalb des Sensorpflasters 126, welcher in der Regel von außen nicht einzusehen ist, überwacht werden, und es können abnormale Zustände beispielsweise erfasst und gegebenenfalls gemeldet bzw. an einen Benutzer 122 übermittelt werden. Dies könnte beispielsweise mittels einer separaten oder im Sensor 110 integrierten optischen und/oder thermischen Sensorik erfolgen. Auch die Daten dieser Sensorik können in den persönlichen Daten enthalten sein und beispielsweise mit an die Steuervorrichtung 142 übermittelt werden. Auf diese Weise kann die Steuervorrichtung 142 beispielsweise entsprechende Warnungen an den Benutzer ausgeben.

### Bezugszeichenliste

- 110: Sensor
- 112: Einweg-Einheit (Disposable)
- 114: Mehrweg-Einheit (Reusable)
- 116: Implantationshilfe
- 118: Sensorelement
- 120: Körpergewebe
- 122: Benutzer
- 124: Schnittstelle
- 126: Sensorpflaster
- 128: Sensoreinheit
- 130: Energiespeicher
- 132: Datenspeicher
- 134: Datenspeicher
- 136: Schnittstelle für drahtlosen Datenaustausch
- 138: Schnittstelle für Zuordnungs- schritt in Mehrwegeinheit
- 140: medizinisches Benutzerele- ment
- 142: Steuervorrichtung
- 144: medizinisches System
- 146: Gehäuse
- 148: Vertiefung
- 150: Zuordnungsschritt
- 152: Schnittstelle für Zuordnungs- schritt
- 154: Schnittstelle für drahtlosen Datenaustausch in Steuervor- richtung
- 156: Koppelelement
- 158: Dockingstation
- 160: Schnittstellen
- 162: Erfassungsvorrichtung
- 164: Erfassungsvorrichtung
- 166: biometrisches Merkmal
- 168: PC-System
- 170: Schnittstelle
- 172: Kurvenzug
- 174: Teil-Datensatz (z.B. logisch)
- 176: Teil-Datensatz (z.B. logisch)
- 178: Teil-Datensatz (z.B. logisch)
- 180: Teil-Datensatz (z.B. physika- lisch)
- 182: Anzeigenelement

## Patentansprüche

1. Medizinisches System (144), insbesondere zur Überwachung und/oder Steuerung mindestens einer Körperfunktion eines Benutzers (122), umfassend eine Steuervorrichtung (142) und mindestens ein von der Steuervorrichtung (142) getrennt ausgebildetes medizinisches Benutzerelement (140), wobei das medizinische Benutzerelement (140) und die Steuervorrichtung (142) eingerichtet sind, um drahtlos Daten auszutauschen, wobei das medizinische System (144) eingerichtet ist, um einen automatischen Zuordnungsschritt zu ermöglichen, wobei durch den automatischen Zuordnungsschritt ein Austausch persönlicher Daten zwischen dem medizinischen Benutzerelement (140) und der Steuervorrichtung (142) freigegeben wird, wobei das medizinische System (144) eingerichtet ist, um den automatischen Zuordnungsschritt durch eine Zuordnungskopplung zwischen dem medizinischen Benutzerelement (140) und der Steuervorrichtung (142) automatisch zu initiieren, wobei das medizinische System (144) eingerichtet ist, um nach dem Zuordnungsschritt eine Trennung der Zuordnungskopplung für einen medizinischen Betrieb des medizinischen Systems (144) zu ermöglichen.

2. Medizinisches System (144) nach dem vorhergehenden Anspruch, wobei die Zuordnungskopplung eine oder mehrere der folgenden Kopplungen zwischen dem medizinischen Benutzerelement (140) und der Steuervorrichtung (142) umfasst:
- eine vorübergehende vorgegebene Kopplungsausrichtung zwischen dem medizinischen Benutzerelement (140) und der Steuervorrichtung (142), wobei die Kopplungsausrichtung von einer Ausrichtung im medizinischen Betrieb verschieden ist;
- eine vorübergehende physikalische Kopplungsverbindung zwischen dem medizinischen Benutzerelement (140) und der Steuervorrichtung (142), insbesondere über eine Kopplungsschnittstelle (138, 152), eine Dockingstation (158), eine drahtgebundene Verbindung, eine drahtlose Nahfeldverbindung oder eine Infrarotverbindung;
- eine indirekte Zuordnungskopplung über mindestens ein Koppelelement (156), wobei das medizinische Benutzerelement (140) und die Steuervorrichtung (142) gleichzeitig oder nacheinander vorübergehend mit dem Koppelelement (156) verbindbar sind.

3. Medizinisches System (144) nach einem der vorhergehenden Ansprüche, wobei das medizinische System (144) eingerichtet ist, um über die Zuordnungskopplung mindestens eine Zuordnungsidentifikation auszutauschen, wobei die Steuervorrichtung (142) vorzugsweise eingerichtet ist, um im medizinischen Betrieb anhand der Zuordnungsidentifikation von dem medizinischen Benutzerelement (140) übermittelte Daten, insbesondere persönliche Daten, diesem medizinischen Benutzerelement (140) zuzuordnen.

4. Medizinisches System (144) nach einem der vorhergehenden Ansprüche, wobei das medizinische Benutzerelement (140) und die Steuervorrichtung (142) in ihrer äußeren Gestalt eingerichtet sind, um eine definierte vorübergehende Kopplungsausrichtung zwischen dem medizinischen Benutzerelement (140) und der Steuervorrichtung (142) zu ermöglichen.

5. Medizinisches System (144) nach dem vorhergehenden Anspruch, wobei die Steuervorrichtung (142) ein Gehäuse (146) aufweist, wobei das Gehäuse (146) mindestens eine einer äußeren Gestalt des medizinischen Benutzerelements (140) entsprechende Vertiefung (148) und/oder Erhöhung aufweist, wobei das medizinische Benutzerelement (140) zur Erzeugung der Zuordnungskopplung vorübergehend in die Vertiefung (148) einbringbar ist und/oder auf die Erhöhung aufbringbar ist.

6. Medizinisches System (144) nach einem der vorhergehenden Ansprüche, wobei die Steuervorrichtung (142) und das medizinische Benutzerelement (140) jeweils eine Erfassungsvorrichtung (162, 164) zur Erfassung eines biometrischen Merkmals (166) des Benutzers (122) aufweisen, wobei die Zuordnungskopplung eine Handhabung des medizinischen Benutzerelements (140) und der Steuervorrichtung (142) durch den Benutzer (122) umfasst, wobei das medizinische System (144) eingerichtet ist, um bei Erkennung desselben biometrischen Merkmals (166) durch die Erfassungsvorrichtungen (162, 164) den Zuordnungsschritt zu initiieren.

7. Medizinisches System (144) nach einem der vorhergehenden Ansprüche, wobei das medizinische System (144) eingerichtet ist, um bei oder nach erfolgreicher Vollendung des Zuordnungsschritts automatisch den medizinischen Betrieb zu starten.

8. Medizinisches System (144) nach einem der vorhergehenden Ansprüche, wobei mehrere medizinische Benutzerelemente (140) vorgesehen sind, wobei die Steuervorrichtung (142) und die medizinischen Benutzerelemente (140) ein medizinisches Netzwerk bilden.

9. Medizinisches System (144) nach dem vorhergehenden Anspruch, wobei das medizinische System (144) eingerichtet ist, um mittels weiterer automatischer Zuordnungsschritte eine automatische Aufnahme weiterer medizinischer Benutzerelemente (140) zu ermöglichen.

10. Medizinisches System (144) nach einem der vorhergehenden Ansprüche, wobei die Steuervorrichtung (142) mindestens eine Datenverarbeitungsvorrichtung umfasst, wobei die Datenverarbeitungsvorrichtung eingerichtet ist, um die persönlichen Daten zumindest teilweise zu verarbeiten.

11. Medizinisches System (144) nach einem der vorhergehenden Ansprüche, wobei die Steuervorrichtung (142) mindestens eine von dem medizinischen Benutzerelement (140) unabhängige Messfunktion aufweist.

12. Medizinisches System (144) nach einem der vorhergehenden Ansprüche, wobei das medizinische Benutzerelement (140) ein oder mehrere der folgenden Elemente umfasst: einen Sensor (110) zum Nachweis mindestens eines Analyten in einer Körperflüssigkeit, insbesondere einen in ein Körpergewebe implantierbaren Sensor (110); eine Messvorrichtung zur Erfassung mindestens einer Körperfunktion; eine Medikationsvorrichtung, insbesondere eine Insulinpumpe; eine medizinische Aktorvorrichtung mit mindestens einem Aktor zur Steuerung einer Körperfunktion.

13. Medizinisches System (144) nach einem der vorhergehenden Ansprüche, wobei das medizinische System (144) weiterhin eingerichtet ist, um einen automatischen Trennschritt zu ermöglichen, wobei durch den automatischen Trennschritt ein weiterer Austausch persönlicher Daten zwischen dem medizinischen Benutzerelement (140) und der Steuervorrichtung (142) verhindert wird.

14. Medizinisches System (144) nach einem der vorhergehenden Ansprüche, wobei das medizinische System (144) eingerichtet ist, um zumindest bei wiederholter kurzfristiger Annäherung zwischen der Steuervorrichtung (142) und dem medizinischen Benutzerelement (140) im medizinischen Betrieb einen wiederholten Austausch komplementärer Datensätze zu ermöglichen.

15. Verfahren zum Betrieb eines medizinischen Systems (144), insbesondere eines medizinischen Systems (144) nach einem der vorhergehenden Ansprüche, wobei eine Steuervorrichtung (142) und mindestens ein medizinisches Benutzerelement (140) verwendet werden, wobei das medizinische Benutzerelement (140) und die Steuervorrichtung (142) drahtlos Daten austauschen, wobei ein automatischer Zuordnungsschritt durchgeführt wird, wobei durch den automatischen Zuordnungsschritt ein Austausch persönlicher Daten zwischen dem medizinischen Benutzerelement (140) und der Steuervorrichtung (142) freigegeben wird, wobei der automatische Zuordnungsschritt durch eine Zuordnungskopplung zwischen dem medizinischen Benutzerelement (140) und der Steuervorrichtung (142) automatisch initiiert wird, wobei die Zuordnungskopplung anschließend für einen medizinischen Betrieb des medizinischen Systems (144) wieder getrennt wird.
